# EUROPEAN PATENT APPLICATION

(11) **EP 3 299 816 A1**
(43) Date of publication of application: **28.03.2018**
(21) Application number: 16796608.4
(22) Date of filing: 20.05.2016
(51) Int. Cl.: G01N 33/543, C12Q 1/68, G01N 33/50, G01N 33/68

(54) **SEPARATION METHOD, DETECTION METHOD, SIGNAL MEASUREMENT METHOD, DISEASE DETERMINATION METHOD, DRUG EFFICACY ASSESSMENT METHOD, KIT, LIQUID COMPOSITION, AND SPECIMEN DILUENT**

(30) Priority: 20.05.2015 JP 2015102515; 01.07.2015 JP 2015133031; 27.01.2016 WO PCT/JP2016/052353
(71) Applicant: JSR Corporation, Tokyo 105-8640 (JP); JSR Life Sciences Corporation, Tokyo 105-8640 (JP)
(72) Inventor: ABE, Hiroki, Tokyo 105-8640 (JP); FUJII, Hiroya, Tokyo 105-8640 (JP); UCHIDA, Kimiko, Tokyo 105-8640 (JP)
(74) Representative: TBK
(86) International application number: PCT/JP2016/065104
(87) International publication number: WO 2016/186215

(57) **Abstract**

Provided is a method capable of reducing non-specific adsorption onto a solid phase carrier, and capable of selectively and efficiently separating a vesicle having a lipid bilayer membrane. A method of separating a vesicle having a lipid bilayer membrane includes: a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier; and a washing step of washing the complex, in which at least any one of the complex forming step and the washing step is performed under an environment having a salt concentration of from 0.15 M to 2 M.

## Description

### Technical Field

The present invention relates to a separation method, a detection method, a signal measurement method, a disease determination method, a drug efficacy evaluation method, a kit, a liquid composition, and a diluent for specimen. More specifically, the present invention relates to a method of separating a vesicle having a lipid bilayer membrane, such as an exosome, a method of detecting a nucleic acid or a protein, a signal measurement method, a disease determination method, a method of evaluating drug efficacy of a drug for disease treatment, and a kit for determining a disease or evaluating drug efficacy, each using the separation method, and to a liquid composition and a diluent for specimen to be used for the separation method.

### Background Art

A vesicle has a structure covered with a lipid bilayer membrane, and among such vesicles, an exosome is known as a vesicular granule which is present in body fluid of a living organism. Similar to a common cell surface, it is known that various membrane proteins are present on a surface of the exosome. Meanwhile, it is also found that microRNA (miRNA) is contained in the inside of the exosome in addition to various proteins, such as cytokines.

It is also known that the exosome is secreted from various cells, such as cells of an immune system or various cancer cells. Attention is given to a function of the exosome as a mediator for intercellular communication in a living organism and also to a relationship between the exosome and a physiological phenomenon or a disease, such as cancer, and investigations have been made thereon. For example, it has already been reported that, when an antibody of EpCAM serving as a tumor marker is used, an exosome is separated from circulating blood of a patient with ovarian cancer and a relationship is found between an expression amount of miRNA derived from the exosome and progress of ovarian cancer (Non Patent Literature 1).

As a four-transmembrane protein expressed on the exosome, there are known CD9, CD63 and CD81 belonging to tetraspanin family. It has been reported in Non Patent Literature 2 that the amount of an exosome is higher in blood plasma from a patient with melanoma as compared to that of a healthy person, and it can be detected and quantified by an antibody against CD63 or an antibody against Caveolin-1 serving as a cancer-related marker. In addition, by combining and reacting a blood plasma sample after centrifugation with anti CD63 antibody or an antibody against various membrane proteins or the like, a signal derived from an exosome of a cancer patient is quantified and analyzed (Patent Literature 1).

As described above, attempts have been made to specifically capture an exosome from a clinical specimen, such as blood serum, blood plasma, or blood, and to use a protein or miRNA contained in the exosome as a novel biomarker for early diagnosis of a disease or detection of recurrence of a disease.

### Citation List

### Patent Literature

[PTL 1] WO 2010/065968 A1

### Non Patent Literature

[NPL 1] D.D. Taylor., et al., Gynecol. Oncol., 110, 13-21 (2008)
[NPL 2] M. Logozzi., et al., PLoS ONE., 4, 1-10 (2009)

### Summary of Invention

### Technical Problem

However, the above-mentioned clinical specimen contains proteins, such as albumins and globulins, at high concentrations, and hence the following significant problem is caused by using the exosome separated from the clinical specimen for determination of a disease: due to non-specific adsorption onto a solid phase carrier or an inner wall of a reaction vessel, a specific reaction, such as an antigen-antibody reaction, is inhibited by a test sample component, or a test sample to be determined as negative is wrongly determined as positive (hereinafter referred to as false positive). The problem is more remarkable when the clinical specimen is blood serum or blood plasma.

In order to reduce the false positives, there has been a used a technique involving, for example, adding a surfactant during a reaction, or overcoating a solid phase carrier with a protein or a polymer. However, there has been a limitation in reducing the false positives due to the non-specific adsorption by only the hitherto used technique. The reduction of the false positives is expected to be an increasingly important task in determination of a disease and evaluation of drug efficacy of a drug for disease treatment each using a vesicle typified by the exosome.

In addition, it has been proposed that a pre-treatment involving, for example, concentrating the exosome by removing a test sample component that may inhibit an antigen-antibody reaction be performed prior to separation of the exosome. As such pre-treatment method, an isolation method based on polyethylene glycol (PEG) precipitation, an isolation method using an ultracentrifuge, or the like has been generally performed. However, even when such pre-treatment is performed, it is difficult to sufficiently reduce the non-specific adsorption, and in addition, such pre-treatment has had a problem of being laborious.

A problem to be solved by the present invention is to provide a method capable of reducing non-specific adsorption onto a solid phase carrier, and capable of selectively and efficiently separating a vesicle having a lipid bilayer membrane. Solution to Problem

In view of the foregoing, the inventors of the present invention conducted extensive investigations. As a result, they found the following. In a method of separating a vesicle having a lipid bilayer membrane comprising a complex forming step of forming a complex of a vesicle having a lipid bilayer membrane and a solid phase carrier to which a ligand which recognizes a surface antigen present on a surface of the vesicle is bound, by mixing a biological sample containing the vesicle with the solid phase carrier to form a complex forming reaction solution, and a washing step of washing the complex by adding a washing solution to the complex of the vesicle and the solid phase carrier, which has been formed in the complex forming step, to form a washing step solution (the complex forming reaction solution and the washing step solution are hereinafter collectively referred to as "reaction solution"), when at least any one of the complex forming step and the washing step is performed under an environment having a salt concentration of from 0.15 M to 2 M in the reaction solution, non-specific adsorption onto the solid phase carrier can be reduced, and the vesicle having a lipid bilayer membrane can be selectively and efficiently separated. Thus, the present invention was completed.

That is, according to the present invention, the following <1> to <23> are provided.

<1> A method of separating a vesicle having a lipid bilayer membrane, comprising: a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier; and a washing step of washing the complex, in which at least any one of the complex forming step and the washing step is performed under an environment having a salt concentration of from 0.15 M to 2 M.
<2> The separation method according to <1>, wherein the complex forming step is performed under an environment having a salt concentration of from 0.15 M to 2 M.
<3> The separation method according to <1> or <2>, wherein the biological sample is body fluid or cell culture supernatant.
<4> The separation method according to any one of <1> to <3>, wherein the biological sample is body fluid.
<5> The separation method according to any one of <1> to <4>, wherein the complex forming step is performed using a complex forming reaction solution containing an inorganic salt.
<6> The separation method according to <5>, wherein the inorganic salt is an alkali metal halide.
<7> The separation method according to any one of <1> to <6>, wherein a complex forming reaction in the complex forming step is performed at a reaction temperature of from 2°C to 42°C.
<8> The separation method according to any one of <1> to <7>, wherein the ligand is an antibody which recognizes a surface antigen present on a surface of the vesicle.
<9> The separation method according to any one of <1> to <8>, wherein the vesicle is an exosome.
<10> The separation method according to any one of <1> to <9>, wherein the solid phase carrier is magnetic particles.
<11> The separation method according to <10>, wherein the washing step is a magnetic collecting step of collecting the magnetic particles by magnetic force and separating the magnetic particles from a liquid phase, and a dispersing step of dispersing the magnetic particles that are separated by the magnetic collecting step in a washing solution.
<12> A method of detecting a nucleic acid in a vesicle, further comprising a nucleic acid detecting step of detecting a nucleic acid in the vesicle after the separation method of any one of <1> to <11>.
<13> A method of detecting a protein derived from a vesicle, further comprising a protein detecting step of detecting a protein present at least one of inside or on a surface of the vesicle after the separation method of any one of <1> to <11>.
<14> A method of measuring a signal derived from a vesicle, further comprising a signal measuring step of measuring an intensity of a signal derived from the vesicle formed to have the complex after the separation method of any one of <1> to <11>.
<15> A method of determining an onset of a disease in a test subject, comprising a step of measuring, based on the signal measurement method of <14>, an intensity of a signal derived from the vesicle formed to have the complex by using a biological sample derived from a test subject.
<16> A method of evaluating drug efficacy of a drug for disease treatment, comprising a step of measuring, based on the signal measurement method of <14>, an intensity of a signal derived from the vesicle formed to have the complex by using a biological sample derived from a test subject before and after administration of a drug for disease treatment.
<17> A kit, comprising: a solid phase carrier to which a ligand which recognizes a surface antigen present on a surface of a vesicle having a lipid bilayer membrane is bound; and a liquid composition containing an inorganic salt or an organic salt and having a salt concentration of 0.15 M or more in terms of content of the inorganic salt or the organic salt.
<18> The kit according to <17>, which is used for determining a disease or evaluating drug efficacy of a drug for disease treatment.
<19> A liquid composition to be used for a method of separating a vesicle having a lipid bilayer membrane, including a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier, and a washing step of washing the complex, for being added in at least any one of the complex forming step and the washing step, the liquid composition having a salt concentration of 0.15 M or more.
<20> A diluent for specimen to be used for a method of separating a vesicle having a lipid bilayer membrane from a biological sample containing the vesicle having a lipid bilayer membrane through use of an insoluble carrier, for forming a complex of the vesicle and the insoluble carrier, the diluent for specimen having a salt concentration of 0.15 M or more.
<21> The diluent for specimen according to <20>, wherein the diluent for specimen comprises an inorganic salt.
<22> The diluent for specimen according to <21>,which is an alkali metal halide.
<23> A method of separating a vesicle having a lipid bilayer membrane from a biological sample containing the vesicle having a lipid bilayer membrane through use of an insoluble carrier, the method comprising using a diluent for specimen having a salt concentration of 0.15 M or more.

### Advantageous Effects of Invention

According to the method of separating a vesicle having a lipid bilayer membrane of the present invention, the non-specific adsorption onto the solid phase carrier can be reduced, and the vesicle having a lipid bilayer membrane can be selectively and efficiently separated.

Therefore, according to the present invention, the nucleic acid in the vesicle, the protein derived from the vesicle, and the signal derived from the vesicle can be simply and accurately detected and measured. In addition, the disease determination method and the drug efficacy evaluation method of the present invention hardly cause false positives. In addition, through use of each of the kit, the liquid composition, and the diluent for specimen of the present invention, the non-specific adsorption onto the solid phase carrier can be reduced, and the vesicle having a lipid bilayer membrane can be selectively and efficiently separated.

### Brief Description of Drawings

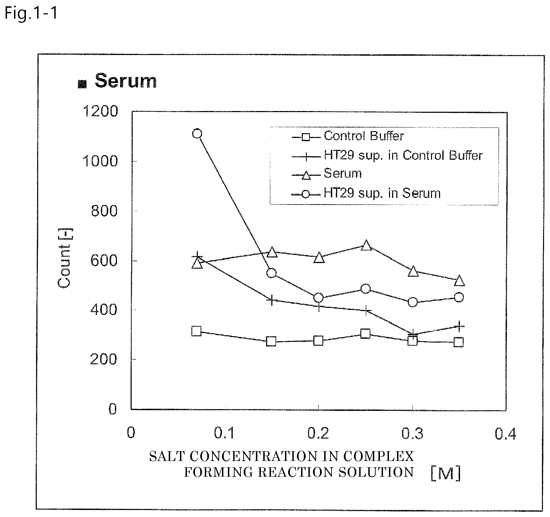

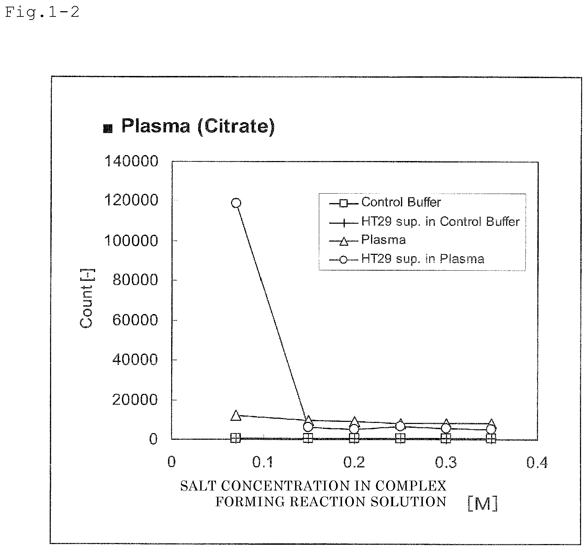

FIG. 1-1 is a graph for showing a non-specific adsorption reducing action at a salt concentration set to from 0.15 M to 0.35 M (clinical specimen: blood serum).
FIG. 1-2 is a graph for showing a non-specific adsorption reducing action at a salt concentration set to from 0.15 M to 0.35 M (clinical specimen: citrated blood plasma).
FIG. 2-1 is a graph for showing a reactivity enhancing action at a salt concentration set to from 0.15 M to 0.35 M at a reaction temperature of 25°C (clinical specimen: blood serum).
FIG. 2-2 is a graph for showing a reactivity enhancing action at a salt concentration set to 0.25 M at a reaction temperature of 4°C (clinical specimen: blood serum).
FIG. 3 is a graph for showing results of investigation of non-specific adsorption reducing actions in the cases of using various clinical specimens.
FIG. 4 is a graph for showing results of investigation of reactivity enhancing actions in the cases of using various clinical specimens.
FIG. 5-1 is a graph for showing results of investigation of a reactivity enhancing action in the case of using anti CD63 antibody bound magnetic particles as a solid phase carrier.
FIG. 5-2 is a graph for showing results of investigation of a reactivity enhancing action in the case of using anti CD81 antibody bound magnetic particles as a solid phase carrier.
FIG. 5-3 is a graph for showing results of investigation of a reactivity enhancing action in the case of using anti EpCAM antibody bound magnetic particles as a solid phase carrier.
FIG. 6 is a graph for showing results of detection of a nucleic acid in a vesicle in the case where anti CD9 antibody bound magnetic particles are used as a solid phase carrier and allowed to react with healthy human blood serum.
FIG. 7-1 is a graph for showing results of detection of a nucleic acid in a vesicle derived from healthy human blood serum.
FIG. 7-2 is a graph for showing results of detection of a nucleic acid in a vesicle derived from healthy human heparin blood plasma.
FIG. 8-1 is an image for showing results of detection of proteins on a surface of a vesicle derived from healthy human blood serum.
FIG. 8-2 is an image for showing results of detection of proteins on a surface of a vesicle derived from healthy human heparin blood plasma.
FIG. 9-1 is a graph for showing results of detection of proteins each maintaining the three-dimensional structure of a native form on a surface of a vesicle derived from healthy human blood serum.
FIG. 9-2 is a graph for showing results of detection of proteins each maintaining the three-dimensional structure of a native form on a surface of a vesicle derived from healthy human heparin blood plasma.
FIG. 9-3 is a graph for showing results of detection of proteins on a surface of a vesicle, each of which still maintains the three-dimensional structure of a native form, from a simulated disease-derived biological sample obtained by adding exosome-containing culture supernatant prepared from human colon cancer HT29 cells to healthy human blood serum.
FIG. 9-4 is a graph for showing results of detection of proteins on a surface of a vesicle, each of which still maintains the three-dimensional structure of a native form, from a simulated disease-derived biological sample obtained by adding exosome-containing culture supernatant prepared from human colon cancer HT29 cells to healthy human heparin blood plasma.
FIG. 10-1 is an image for showing results of detection of proteins on a surface of a vesicle from a simulated disease-derived biological sample obtained by adding exosome-containing culture supernatant prepared from human colon cancer HT29 cells to healthy human blood serum.
FIG. 10-2 is an image for showing results of detection of proteins on a surface of a vesicle from a simulated disease-derived biological sample obtained by adding exosome-containing culture supernatant prepared from human colon cancer HT29 cells to healthy human heparin blood plasma. Description of Embodiments

### [Method of Separating Vesicle Having Lipid Bilayer Membrane]

A method of separating a vesicle having a lipid bilayer membrane of the present invention comprises: a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier; and a washing step of washing the complex, in which at least any one of the complex forming step and the washing step is performed under an environment having a salt concentration of from 0.15 M to 2 M. As described herein, the salt concentration refers to the total salt concentration of an inorganic salt and an organic salt, preferably the salt concentration of an inorganic salt, more preferably the salt concentration of a monovalent inorganic salt, even more preferably the concentration of an alkali metal halide, most preferably the concentration of sodium chloride. The salt concentration of a reaction solution is also referred to as final concentration of a salt in the reaction solution.

### (Complex Forming Step)

The complex forming step is a step of forming a complex of a vesicle having a lipid bilayer membrane and a solid phase carrier to which a ligand which recognizes a surface antigen present on a surface of the vesicle is bound, by mixing a biological sample containing the vesicle with the solid phase carrier to form a complex forming reaction solution. Through the formation of the complex forming reaction solution, the biological sample containing the vesicle having a lipid bilayer membrane is brought into contact with the solid phase carrier to which a ligand which recognizes a surface antigen present on a surface of the vesicle is bound. Through the contact, the vesicle is captured by the ligand, and the complex of the vesicle and the solid phase carrier is formed. In a reaction system for the complex forming step, a ligand which recognizes a surface antigen present on a surface of the vesicle having a lipid bilayer membrane other than the ligand bound to the solid phase carrier may also be present.

The biological sample is not particularly limited as long as the biological sample contains a vesicle having a lipid bilayer membrane, and examples thereof include various liquids, such as body fluid, bacterial liquid, cell culture medium, cell culture supernatant, and liquid containing disrupted tissue cells. Of those, body fluid and cell culture supernatant are preferred, and body fluid is more preferred. Examples of the body fluid include components of blood composition, such as whole blood, blood serum, blood plasma, blood components, various blood cells, blood clot, and blood platelet, and also urine, semen, milk, sweat, interstitial fluid, interstitial lymph, bone marrow fluid, tissue fluid, saliva, gastric juice, joint fluid, pleural fluid, bile acid, ascite, and amniotic fluid. Of those, components of blood composition are preferred. In the separation method of the present invention, even when such clinical specimen is used as the biological sample, non-specific adsorption onto the solid phase carrier is low and the reactivity of a capturing reaction is high. Accordingly, according to the separation method of the present invention, the vesicle can be separated selectively and efficiently from a wide variety of biological samples. For example, even when blood plasma is used as the biological sample, non-specific adsorption hardly occurs, and even when blood serum is used, the reactivity of the capturing reaction is high.

The components of blood composition may be treated with an anti-clotting agent, such as citric acid, heparin, or EDTA.

The biological sample may be used as a pre-treated sample after being, for example, diluted with a buffer composition in advance. Alternatively, a sample collected from a living organism may be used as it is. That is, according to the separation method of the present invention, a simple and selective separation can be made without performing a pre-treatment based on an isolation method or the like using PEG precipitation, an ultracentrifuge, or the like.

In addition, although examples of the vesicle having a lipid bilayer membrane include a cell, a vesicle such as an exosome which is discharged extracellularly from a cell, and a virus having an envelope, the separation method of the present invention is more preferably used for a case in which the vesicle is an exosome.

In addition, the surface antigen present on a surface of the vesicle is not particularly limited as long as the surface antigen is a substance present on a vesicle surface and having antigenicity. A surface antigen of an exosome is exemplified by: tetraspanins, such as CD9, CD63, and CD81; proteins related to antigen-presentation, such as MHCI and MHCII; adhesion molecules, such as integrin, ICAM-1, and EpCAM; and cytokines, such as EGFRvIII and TGF-β/cytokine receptors, and enzymes. Of those, an antigen protein present on an exosome surface is preferred.

The used amount (concentration) of the biological sample is preferably from 0.001% (w/v) to 100% (w/v), more preferably from 0.1% (w/v) to 50% (w/v).

In addition, the solid phase carrier to be used for the separation method of the present invention is not particularly limited as long as the solid phase carrier has bound thereto a ligand which recognizes a surface antigen present on a surface of a vesicle.

The ligand is preferably an antibody which recognizes a surface antigen present on a surface of a vesicle, more preferably an antibody which recognizes a surface antigen present on a surface of an exosome. In addition, the ligand may be a monoclonal antibody or a polyclonal antibody, and is preferably a monoclonal antibody.

The monoclonal antibody is not particularly limited, and may be an antibody produced in accordance with a known method, for example, a method described in K. Watanabe et al., Vasohibin as an endothelium-derived negative feedback regulator of angiogenesis, J. Clin. Invest. 114 (2004), 898-907. In addition, a monoclonal antibody which recognizes any of tetraspanins, such as surface antigens CD9, CD63, and CD81 of an exosome, may be prepared with reference to WO 2013/099925 A1 or the like.

Examples of the class of the antibody include IgG and IgM. Of those, IgG is preferred. In addition, a fragment obtained by decomposing any such antibody into a small molecule may also be used. Examples thereof include F(ab')2, Fab', and Fab.

A material of the solid phase carrier for binding the above-mentioned ligand is exemplified by: a high molecular compound, such as polystyrenes, polyethylenes, polypropylenes, polyesters, poly(meth)acrylonitriles, a styrene-butadiene copolymer, poly(meth)acrylic acid esters, a fluororesin, cross-linked dextran, or polysaccharides; glass; a metal; a magnetic material; a resin composition containing a magnetic material; and a combination thereof.

In addition, the shape of the solid phase carrier is not particularly limited, and examples thereof include a tray, a globule, a particle, a fiber, a rod, a dish, a container, a cell, a micro plate, and a test tube.

In the present invention, from the viewpoint of easiness in solid-liquid separation or washing, magnetic particles are preferred.

Examples of the magnetic particle include: a metal, such as ferric oxide (Fe₃O₄), maghemite (γ-Fe₂O₃), various ferrites, iron, manganese, nickel, cobalt, or chromium; magnetic fine particles formed of an alloy of, for example, cobalt, nickel, or manganese; and magnetic particles containing those magnetic materials in a resin. Examples of the resin include a hydrophobic polymer and a hydrophilic polymer.

Of those, magnetic particles each containing a magnetic material in a resin are preferred, and magnetic particles each obtained by forming a polymer layer on a surface of a parent particle containing superparamagnetic fine particles are more preferred. Examples thereof include magnetic particles described in JP-A-2008-32411 each obtained by forming a hydrophobic first polymer layer on a surface of a parent particle containing superparamagnetic fine particles, forming, on the first polymer layer, a second polymer layer having a glycidyl group at least on a surface thereof, and introducing a polar group therein by chemical modification of the glycidyl group.

Typical examples of the superparamagnetic fine particles include fine particles of iron oxide each having a particle diameter of 20 nm or less (preferably a particle diameter of from 5 nm to 20 nm), and examples thereof include ferrite represented by XFe₂O₄ (X represents Mn, Co, Ni, Mg, Cu, Li_{0.5}Fe_{0.5}, or the like), magnetite represented by Fe₃O₄, and γ-Fe₂O₃. From the viewpoint of having strong saturation magnetization and little residual magnetization, the superparamagnetic fine particles each preferably contain any one of γ-Fe₂O₃ and Fe₃O₄.

In addition, monomers for forming the hydrophobic first polymer layer are roughly classified into a monofunctional monomer and a polyfunctional monomer. Herein, the monofunctional monomer refers to a monomer having one reactive group, and the polyfunctional monomer refers to a monomer having two or more reactive groups.

Examples of the monofunctional monomer include: an aromatic vinyl monomer, such as styrene, α-methylstyrene, or halogenated styrene; and an ethylenically unsaturated carboxylic acid alkyl ester monomer, such as methyl acrylate, methyl methacrylate, ethyl acrylate, ethyl methacrylate, stearyl acrylate, stearyl methacrylate, cyclohexyl acrylate, cyclohexyl methacrylate, isobornyl acrylate, or isobornyl methacrylate.

Examples of the polyfunctional monomer include: a polyfunctional (meth)acrylate, such as ethylene glycol diacrylate, ethylene glycol dimethacrylate, trimethylol propane triacrylate, trimethylol propane trimethacrylate, pentaerythritol triacrylate, pentaerythritol trimethacrylate, dipentaerythritol hexaacrylate, or dipentaerythritol hexamethacrylate; a conjugated diolefin, such as butadiene or isoprene; and divinylbenzene, diallyl phthalate, allyl acrylate, and allyl methacrylate.

In addition, monomers for forming the second polymer layer are mainly intended to introduce a functional group to a particle surface, and preferably include a monomer containing a glycidyl group. The content of the monomer containing a glycidyl group is preferably 20% by mass or more in all the monomers for forming the second polymer layer. Examples of the monomer containing a glycidyl group include glycidyl acrylate, glycidyl methacrylate, and allyl glycidyl ether.

The polar group to be introduced by chemical modification of the glycidyl group in the second polymer layer is preferably a functional group which can react with a ligand, more preferably a group containing one or more of at least one kind of atom selected from the group consisting of an oxygen atom, a nitrogen atom, and a sulfur atom. Of those, an amino group, an aldehyde group, a carboxy group, and an active ester group are more preferred. Particularly when the second polymer layer of each of the magnetic particles contains the polar group and a 2,3-dihydroxypropyl group, a good binding property with a ligand is obtained.

In addition, the solid phase carrier, such as the magnetic particles, may have immobilized on a surface thereof a substance having a binding site for biotin or an analog thereof. Examples of the substance having a binding site for biotin or an analog thereof include one kind or two or more kinds of compounds selected from avidin, streptavidin, tamavidin, and derivatives thereof. Further, in this case, the solid phase carrier may have bound thereto an antibody or the like bound with biotin or an analog thereof, via the substance having a binding site for biotin or an analog thereof. The immobilization of the substance having a binding site for biotin or an analog thereof onto the surface of the solid phase carrier may be performed by a known method, such as a method described in JP-B-4716034.

As a method of binding the ligand to the solid phase carrier, for example, a physical adsorption method or a chemical binding method, such as a covalent bond method or an ionic bond method, is used. Examples of the physical adsorption method include a method involving directly immobilizing the ligand onto the solid phase carrier and a method involving chemically binding the ligand to another protein, such as albumin, followed by immobilization based on adsorption. Examples of the chemical binding method include a method involving directly binding the ligand onto the solid phase carrier by using a functional group introduced to the surface of the solid phase carrier, in which the functional group is capable of reacting with the ligand, a method involving carrying out binding after introduction of a spacer molecule (for example, carbodiimide compound) between the solid phase carrier and the ligand by chemical binding, and a method involving binding the ligand to another protein, such as albumin, followed by chemical binding of the protein to the solid phase carrier.

In addition, a commercially available product may be used as the solid phase carrier to which a ligand which recognizes a surface antigen present on a surface of the vesicle is bound. Examples thereof include Exosome-Human CD9 Isolation Reagent (from cell culture) and Exosome-Human CD63 Isolation/Detection Reagent (from cell culture media) (each of which is manufactured by Life Technologies Corporation), and CD9 Exo-Flow capture kit, CD63 Exo-Flow capture kit, and CD81 Exo-Flow capture kit (all of which are manufactured by SBI).

In addition, the used amount of the solid phase carrier to which the ligand is bound is preferably from 0.00001 times by mass to 0.1 times by mass, more preferably from 0.0001 times by mass to 0.01 times by mass, with respect to the biological sample.

In the complex forming reaction solution in the complex forming step, in addition to each component described above, as necessary, there may also be added: a surfactant; a protein, such as albumin; a nucleic acid; a saccharide, such as sucrose; or the like.

From the viewpoints of a reduction in non-specific adsorption and effectiveness in suppressing damage to the vesicle, the surfactant is preferably a nonionic surfactant, more preferably a polyethylene glycol type nonionic surfactant. In addition, the surfactant is preferably a surfactant not containing an aromatic group in the molecule.

Examples of the surfactant not containing an aromatic group in the molecule include higher alcohol ethylene oxide adducts, fatty acid ethylene oxide adducts, polyhydric alcohol fatty acid ester ethylene oxide adducts, higher alkylamine ethylene oxide adducts, fatty acid amide ethylene oxide adducts, ethylene oxide adducts of oils and fats, and polyalkylene glycol ethylene oxide adducts. Of those, polyalkylene glycol ethylene oxide adducts are preferred. Specific examples thereof include polyoxyethylene (160) polyoxypropylene (30) glycol and polyoxyethylene (196) polyoxypropylene (67) glycol.

The used amount of the surfactant is preferably from 0.005% (w/v) to 10% (w/v), more preferably from 0.015% (w/v) to 3.5% (w/v), with respect to the entire amount of the complex forming reaction solution.

In addition, the reaction temperature in the complex forming step falls within preferably the range of from 2°C to 42°C, more preferably the range of from 3°C to 40°C, even more preferably the range of from 4°C to 37°C. The complex forming step in the separation method of the present invention is extremely simple because the reaction proceeds selectively and efficiently even at normal temperature or around normal temperature. In addition, the reaction time is generally from 1 minute to 48 hours, and is preferably from 5 minutes to 24 hours, more preferably from 10 minutes to 10 hours, even more preferably from 10 minutes to 5 hours.

In the complex forming step, the pH in the system is not particularly limited, but the pH preferably falls within the range of from 5 to 10, and the pH more preferably falls within the range of from 6 to 8. In order to maintain a desired pH, a buffer solution is generally used. Examples of the buffer solution include a phosphate buffer solution, a tris(hydroxymethyl)aminomethane buffer solution, a HEPES buffer solution, and an MES buffer solution.

### (Washing Step)

The washing step is a step of washing the complex of the vesicle and the solid phase carrier, which has been formed in the complex forming step, by adding a washing solution to the complex to form a washing step solution. According to this step, an unreacted component, an unreacted labeling material, and the like are removed. A system containing the complex of the complex forming step may be directly washed.

The washing step is generally classified into two types depending on the shape of the solid phase carrier. When the solid phase carrier has a particle shape like magnetic particles, a washing method involving dispersing magnetic particles in the washing solution may be given as an example. Meanwhile, when the solid phase carrier has a shape like a micro plate, a washing method involving bringing the surface thereof into contact with the washing solution may be given as an example. For any of those embodiments, it is preferred in the present invention to use, as the washing solution, a washing solution containing a surfactant. The surfactant is preferably one similar to the one that may be used in the complex forming step.

In addition, when the solid phase carrier is magnetic particles, the washing step preferably includes a magnetic collecting step of collecting the magnetic particles by magnetic force and separating the magnetic particles from a liquid phase, and a dispersing step of dispersing the magnetic particles that are separated by the magnetic collecting step in a washing solution. With this, unreacted substances or impurities in the biological sample can be more efficiently washed off, and separated and removed from the surfaces of the magnetic particles.

Specifically, the washing step may be performed by: collecting the magnetic particles by applying a magnetic field to a reaction vessel to cause the magnetic particles to adhere to a wall of the reaction vessel; then removing the reaction supernatant; adding the washing solution as necessary; and repeating an operation of similarly applying a magnetic field and removing the supernatant. The washing solution is preferably a washing solution which contains the surfactant and buffer solution described in the complex forming step.

### (Salt Concentration of Reaction Solution)

In the separation method of the present invention, at least any one of the complex forming step and the washing step is preferably performed under an environment having a salt concentration of from 0.15 M to 2 M. That is, an environment in which at least any one of the salt concentration of the complex forming reaction solution and the salt concentration of the washing step solution is from 0.15 M to 2 M is preferred. Further, from the viewpoint of the reactivity of the capturing reaction, it is preferred that the complex forming step be performed under an environment having a salt concentration of from 0.15 M to 2 M. The salt concentration of the reaction solution only needs to fall within the range of from 0.15 M to 2 M in the entirety or part of the complex forming step, or in the entirety or part of the washing step.

The salt concentration of the reaction solution is more preferably 0.2 M or more, still more preferably 0.25 M or more, and is more preferably 1 M or less, still more preferably 0.5 M or less, even more preferably 0.45 M or less, most preferably 0.35 M or less.

Examples of the inorganic salt to be contained in the reaction solution include: an alkali metal halide; and an inorganic acid salt, such as a carbonate or a bicarbonate. Of those, an alkali metal halide is preferred, and an alkali metal chloride is more preferred. Specific examples of the inorganic salt include sodium chloride, potassium chloride, calcium chloride, sodium carbonate, and sodium bicarbonate, and those inorganic salts may be used alone or in combination thereof. Of those, sodium chloride, potassium chloride, and calcium chloride are preferred, sodium chloride and potassium chloride are more preferred, and sodium chloride is even more preferred.

As the organic salt to be contained in the reaction solution, an organic acid salt is preferred, an alkali metal salt of an organic acid is more preferred, an alkali metal salt of a carboxylic acid is still more preferred, and a sodium salt of a carboxylic acid is even more preferred. Specific examples of the organic salt include sodium acetate, sodium citrate, sodium tartrate, sodium malate, sodium succinate, and sodium lactate, and those organic salts may be used alone or in combination thereof.

In each of the case where the salt concentration of the reaction solution is less than 0.15 M and the case where the salt concentration is more than 2 M, an increase in non-specific adsorption or a decrease in reactivity of the capturing reaction may occur, and in addition, the vesicle may be disrupted owing to a difference in osmotic pressure between the inside and outside of the vesicle.

### (Dissociating Step)

The separation method of the present invention may also comprise, after the washing step, a dissociating step of dissociating the captured vesicle from the ligand.

As the condition for dissociation of a specific bond based on an antigen/antibody reaction, for a case in which the ligand is a specific antibody, various conditions have been known for affinity chromatography or the like (see, for example, "Afinity Chromatography Principles & Methods" Pharmacia LKB Biotechnology). The dissociation step may be performed in accordance with the condition. That is, a dissociating solution to be used in the present invention is exemplified by: an acidic solution, such as hydrochloric acid, sulfuric acid, propionic acid, acetic acid, or glycine/hydrochloric acid buffer; an alkali solution, such as an aqueous sodium hydroxide solution, an aqueous potassium hydroxide solution, an aqueous ammonia solution, or diethylamine; a high ionic strength solution, such as a 3 M aqueous solution of sodium chloride or a 4.5 M aqueous solution of magnesium chloride; a solution containing a surfactant, such as SDS, Triton X-100, or Tween 20; a buffer solution containing a substance for lowering polarity, such as dioxane or ethylene glycol; and a buffer solution containing urea, guanidine hydrochloride, or the like, in addition to chaotropic ions, such as trichloroacetic acid or thiocyanic acid.

### [Method of Detecting Nucleic Acid and Method of Detecting Protein]

A method of detecting a nucleic acid in a vesicle of the present invention further comprises a nucleic acid detecting step of detecting a nucleic acid in the vesicle after the above-mentioned separation method.

In addition, a method of detecting a protein derived from a vesicle of the present invention further comprises a protein detecting step of detecting a protein present at least one of inside or on a surface of the vesicle after the above-mentioned separation method.

Those detection methods may each be performed in accordance with a normal method except for performing the above-mentioned separation method. When the vesicle having a lipid bilayer membrane is an exosome, the nucleic acid or the protein may be detected from a recovered exosome by a known method, such as a PCR method, an electrophoretic method, a western blot method, an immunochemical method, and flow cytometry. Of those, from the viewpoints of allowing detection to be performed simply and with good sensitivity, and allowing the three-dimensional structure of a native form to be maintained, flow cytometry is preferred. In addition, examples of the nucleic acid include miRNA and mRNA.

In particular, as the exosome is secreted from various cells, for example, cells of an immune system or various cancer cells, a nucleic acid (miRNA or the like) derived from an exosome can be detected by the nucleic acid detection method of the present invention, and by the analysis thereof, a physiological phenomenon or various diseases can be determined.

### [Signal Measurement Method]

A method of measuring a signal derived from a vesicle of the present invention further comprises a signal measuring step of measuring an intensity of a signal derived from the vesicle formed to have the complex after the above-mentioned separation method.

The measurement of the intensity of the signal may be performed by an immunological measurement method, and examples thereof include enzyme immunoassay (EIA), an enzyme immunometric assay method (ELISA), fluorescent immunoassay (FIA), radioactive immunoassay (RIA), luminescence immunoassay, an immuno blotting method, a western blot method, and flow cytometry. Of those, from the viewpoints of allowing detection to be performed simply and with good sensitivity, and allowing the three-dimensional structure of a native form to be maintained, flow cytometry is preferred. In addition, from the viewpoint of allowing an antibody to be detected simply and with good sensitivity, an ELISA method is preferred. Examples of the ELISA method include a competition method and a sandwich method. In addition, from the viewpoint of detection sensitivity, luminescence immunoassay is also preferred. The luminescence immunoassay may be performed by, for example, subjecting a commercially available luminescent substrate, for example, Class III Series manufactured by Fujirebio Inc., to a reaction, and measuring a luminescence intensity, which is the intensity of the signal in the luminescence immunoassay, through use of a luminescence measuring device, for example, GloMax manufactured by Promega.

Now, an exemplary separation method for the case of using a sandwich ELISA method is described. First, a ligand which recognizes a surface antigen present on a vesicle surface is allowed to bind to a solid phase carrier, and then the resultant is brought into contact with a biological sample containing a vesicle having a lipid bilayer membrane to form a complex, followed by washing. A labeled antibody obtained by modifying a monoclonal antibody or an antibody fragment thereof, or a disease-specific membrane protein antibody or an antibody fragment thereof is further added to form an additional complex. Further, a label amount in the formed complex is detected. Thus, the amount of a signal derived from a vesicle contained in the biological sample or the amount of a disease-specific vesicle contained in the biological sample can be measured.

### [Disease Determination Method]

A method of determining an onset of a disease in a test subject of the present invention comprises a step of measuring, based on the above-mentioned signal measurement method, an intensity of a signal derived from the vesicle formed to have the complex by using a biological sample derived from a test subject (hereinafter sometimes referred to as step (I)).

Further, when the intensity of the signal measured in the step (I) is compared to the intensity of a signal of a biological sample derived from a control and the intensity of the signal from the test subject is recognized to be stronger than the intensity of the signal from the control, it may be determined that there is an onset of the disease in the test subject (hereinafter sometimes referred to as step (II)).

Examples of the disease which can be determined by the disease determination method of the present invention include a cancer disease (for example, colon cancer, breast cancer, endometrial cancer, cervical cancer, ovarian cancer, pancreas cancer, stomach cancer, esophageal cancer, liver cancer, lung cancer, kidney cancer, or skin cancer), an inflammation system related disease (for example, rheumatism, arthrosis deformans, nephropathy, pancreatitis, hepatitis, or allergic response), a neurodegenerative disease, such as Alzheimer disease, a brain disease, a disease related to immunodeficiency, infertility, a mental disease, such as depression or autism, an intractable disease, such as Parkinson's disease, an autoimmune disease, a circulatory system disease, a blood disease, a digestive system disease, a senile disease, and an infectious disease. Of those, the method is useful for determination of a cancer disease and a disease of an immunological system, and is particularly useful for determination of a cancer disease.

In addition, when applied to an index of immune activity of cytotoxic T cells (CTL), the method can be used for determination of an effect of a cancer vaccine on a cancer disease.

### The step (I) may be performed by a normal method except for performing the measurement by the above-mentioned signal measurement method.

In addition, in the step (II), comparison is made by performing a statistical analysis based on the signal of the biological sample derived from the control with respect to the intensity of the signal measured in the step (I). The analysis method is not particularly limited, and a known method may be used. In addition, in the determination thereafter, for example, when the signal of the biological sample derived from the test subject is stronger than the signal from the control, it is determined that there is a high possibility of having an onset of the disease. In the present invention, the control means an average person who belongs to the same age group of the same sex as the test subject and exhibits no onset of the disease. The intensity of the signal from the control may be measured at the same time with the intensity of the signal from the test subject, or a statistical value obtained from values that have been separately measured in advance may be used.

### [Method of Evaluating Drug Efficacy of Drug for Disease Treatment]

A method of evaluating drug efficacy of a drug for disease treatment of the present invention comprises a step of measuring, based on the above-mentioned signal measurement method, an intensity of a signal derived from the vesicle formed to have the complex by using a biological sample derived from a test subject before and after administration of a drug for disease treatment (hereinafter sometimes referred to as step (A)).

Further, when the intensity of the signal derived from the complex in the biological sample derived from the test subject after administration of the drug for disease treatment is recognized to be weaker than the intensity of the signal derived from the complex in a biological sample derived from the test subject before administration of the drug for disease treatment, it may be determined that there is a high possibility that the drug for disease treatment exhibits drug efficacy (hereinafter sometimes referred to as step (B)).

An example of the drug for disease treatment is a drug for treating a disease that can be determined by the above-mentioned determination method. Preferred specific examples thereof include an anti-cancer drug and a drug for an anti-immunological system related disease.

The step (A) may be performed by a normal method except for performing the measurement by the above-mentioned signal measurement method.

In addition, in the step (B), comparison is made by performing a statistical analysis based on the signal of the biological sample before administration of the drug for disease treatment with respect to the intensity of the signal measured in the step (A). The analysis method is not particularly limited, and a known method may be used. In addition, in the determination thereafter, for example, when a signal amount of the biological sample after administration of the drug for disease treatment is less than a signal amount of the biological sample before the administration, it is determined that the drug is highly likely to have an effect of inhibiting the disease.

In particular, as an exosome is secreted from various cells, for example, cells of an immune system or various cancer cells, it is believed that drug efficacy in a patient can be evaluated by measuring a change in a blood exosome before and after the administration of the drug for disease treatment (including a change in amount of a membrane protein in addition to an increase/decrease in presence amount of the exosome). In addition, for example, when a ligand for a membrane protein which is specific to a cancer cell is combined with a ligand for a surface antigen of an exosome, it is expected that, for example, specificity of cancer diagnosis can be improved or identification of cancer type can be made, and thus development of a diagnostic drug more specific to a cancer disease can be achieved.

### [Kit]

A kit of the present invention comprises: a solid phase carrier to which a ligand which recognizes a surface antigen present on a surface of a vesicle having a lipid bilayer membrane is bound; and a liquid composition having a salt concentration of 0.15 M or more. Such kit is useful for the above-mentioned separation method, determination of a disease, and evaluation of drug efficacy of a drug for disease treatment.

The solid phase carrier and an inorganic salt or an organic salt to be contained in the liquid composition may be similar to those used for the above-mentioned separation method.

The salt concentration in the liquid composition is preferably 0.3 M or more, more preferably 0.4 M or more, still more preferably 0.5 M or more, and is preferably 4 M or less, more preferably 2 M or less, still more preferably 1 M or less, still more preferably 0.9 M or less, even more preferably 0.7 M or less.

In addition, the liquid composition may contain a surfactant similar to that in the above-mentioned separation method. In addition, the pH of the liquid composition is not particularly limited, but the pH preferably falls within the range from from 5 to 10, and the pH more preferably falls within the range of from 6 to 8. In order to maintain a desired pH, a buffer solution is generally used, and examples thereof include a phosphate buffer solution, a tris(hydroxymethyl)aminomethane buffer solution, a HEPES buffer solution, and an MES buffer solution.

### [Liquid Composition and Diluent for specimen]

A liquid composition of the present invention is a liquid composition to be used for a method of separating a vesicle having a lipid bilayer membrane including a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier, and a washing step of washing the complex, for being added in at least any one of the complex forming step and the washing step, the liquid composition having a salt concentration of 0.15 M or more. In addition, a diluent for specimen of the present invention is a diluent for specimen to be used for a method of separating a vesicle having a lipid bilayer membrane from a biological sample containing the vesicle having a lipid bilayer membrane through use of an insoluble carrier, for forming a complex of the vesicle and the insoluble carrier, the diluent for specimen having a salt concentration of 0.15 M or more.

### [Examples]

The present invention is hereinafter described in detail by way of specific examples. The present invention is not limited to these specific examples.

### (Preparation of Buffers)

A comparative reaction buffer 1 and reaction buffers 1 to 5 shown in Table 1 were prepared.

Reaction buffers: buffers each obtained by adding, to Tris buffered saline (TBS (composition was as follows: 1,370 mmol/L NaCl, 26.8 mmol/L KCl, and 250 mmol/L Tris; a dilution of 10× TBS manufactured by Sigma-Aldrich with water by a factor of 10. pH 7.4. The same applies hereinafter.)), polyoxyethylene (160) polyoxypropylene (30) glycol (Pluronic F-68 manufactured by Gibco, the same applies hereinafter) serving as a nonionic surfactant at 0.03% (w/v), and further adding, as necessary, sodium chloride (manufactured by Wako Pure Chemical Industries, Ltd., JIS Special Grade) at a concentration shown in Table 1.

**Table 1**

| | Inorganic salt |
|---|---|
| Comparative reaction buffer 1 | NaCl |
| | 0.14 M |
| Reaction buffer 1 | NaCl |
| | 0.3 M |
| Reaction buffer 2 | NaCl |
| | 0.4 M |
| Reaction buffer 3 | NaCl |
| | 0.5 M |
| Reaction buffer 4 | NaCl |
| | 0.6 M |
| Reaction buffer 5 | NaCl |
| | 0.7 M |

The following buffer was prepared as a control buffer.

Control buffer: a buffer obtained by adding, to TBS, polyoxyethylene (160) polyoxypropylene (30) glycol serving as a nonionic surfactant at a concentration of 0.03% (w/v). pH 7.4.

### (Preparation of Test Samples)

Test sample 1 (HT29 sup. in Control Buffer) : a test sample obtained by adding, to the control buffer, a 100× concentrate of HT29 cell culture supernatant (without concentration of a low-molecular-weight salt. The same applies hereinafter.) at a dilution factor of 20.
Test sample 2 (Serum): pooled blood serum (manufactured by Kohjin Bio Co., Ltd.).
Test sample 3 (HT29 sup. in Serum) : a test sample obtained by adding, to pooled blood serum (manufactured by Kohj in Bio Co., Ltd.), a 100× concentrate of HT29 cell culture supernatant at a dilution factor of 20.
Test sample 4 (Plasma): pooled citrated blood plasma (manufactured by Kohjin Bio Co., Ltd.).
Test sample 5 (HT29 sup. in Plasma) : a test sample obtained by adding, to pooled citrated blood plasma (manufactured by Kohjin Bio Co., Ltd.), a 100× concentrate of HT29 cell culture supernatant at a dilution factor of 20.
Test sample 6 (HT29 sup. in Control Buffer) : a test sample obtained by adding, to the control buffer produced in Test Example 1, a 100× concentrate of HT29 cell culture supernatant at a dilution factor of 100.
Test sample 7 (Serum): pooled blood serum (manufactured by Kohjin Bio Co., Ltd.).
Test sample 8 (HT29 sup. in Serum) : a test sample obtained by adding, to pooled blood serum (manufactured by Kohj in Bio Co., Ltd.), a 100× concentrate of HT29 cell culture supernatant at a dilution factor of 100.
Test sample 9 (HT29 sup. spike Control Buffer): a test sample obtained by adding, to the control buffer produced in Test Example 1, a 100× concentrate of HT29 cell culture supernatant at a dilution factor of 100.
Test sample 10 (Serum): pooled blood serum (manufactured by Kohjin Bio Co., Ltd.).
Test sample 11 (HT29 sup. spike Serum): a test sample obtained by adding, to pooled blood serum (manufactured by Kohj in Bio Co., Ltd.), a 100× concentrate of HT29 cell culture supernatant at a dilution factor of 100.
Test sample 12 (Plasma (Heparin)): pooled heparin blood plasma (manufactured by Kohjin Bio Co., Ltd.).
Test sample 13 (HT29 sup. spike Plasma (Heparin)): a test sample obtained by adding, to pooled heparin blood plasma (manufactured by Kohjin Bio Co., Ltd.), a 100× concentrate of HT29 cell culture supernatant at a dilution factor of 100.
Test sample 14 (Plasma (EDTA)): pooled EDTA blood plasma (manufactured by Kohjin Bio Co., Ltd.).
Test sample 15 (HT29 sup. spike Plasma (EDTA)): a test sample obtained by adding, to pooled EDTA blood plasma (manufactured by Kohjin Bio Co., Ltd.), a 100× concentrate of HT29 cell culture supernatant at a dilution factor of 100.
Test sample 16 (Plasma (Citrate)): pooled citrated blood plasma (manufactured by Kohjin Bio Co., Ltd.).
Test sample 17 (HT29 sup. spike Plasma (Citrate)): a test sample obtained by adding, to pooled citrated blood plasma (manufactured by Kohjin Bio Co., Ltd.), a 100× concentrate of HT29 cell culture supernatant at a dilution factor of 100.
Test sample 18 (HT29 sup. spike Serum): a test sample obtained by adding, to pooled blood serum (manufactured by Kohj in Bio Co., Ltd.), a 100× concentrate of HT29 cell culture supernatant at a dilution factor of 10.
Test sample 19 (HT29 sup. spike Plasma (Heparin)): a test sample obtained by adding, to pooled heparin blood plasma (manufactured by Kohjin Bio Co., Ltd.), a 100× concentrate of HT29 cell culture supernatant at a dilution factor of 10.

### (Preparation of Antibody Bound Particles)

Negative control particles: particles obtained by binding anti mouse IgG antibody (manufactured by JSR Life Sciences Corporation, the same applies hereinafter) to magnetic particles (MS300/Carboxyl manufactured by JSR Life Sciences Corporation, the same applies hereinafter.). The negative control particles do not react with an exosome.

Anti CD9 antibody bound magnetic particles: particles obtained by binding CD9 antibody (ab2215 manufactured by Abcam plc, the same applies hereinafter) to magnetic particles.

Anti CD63 antibody bound magnetic particles: particles obtained by binding CD63 antibody (manufactured by JSR Life Sciences Corporation) to magnetic particles.

Anti CD81 antibody bound magnetic particles: particles obtained by binding anti CD81 antibody (Clone M38, Abnova MAB6435) to magnetic particles.

Anti EpCAM antibody bound magnetic particles: particles obtained by binding anti EpCAM antibody (manufactured by JSR Life Sciences Corporation) to magnetic particles.

Composite antibody bound magnetic particles: particles obtained by mixing the anti CD9 antibody bound magnetic particles, the anti CD63 antibody bound magnetic particles, the anti CD81 antibody bound magnetic particles, and the anti EpCAM antibody bound magnetic particles at a ratio of 1:1:1:1.

### (Test Example 1)

To a 96 well white plate (#23711007, manufactured by Corning Incorporated), 0.0125 mg of the negative control particles were added, and to each of the wells, 25 µL of the control buffer or any one of the test samples 1 to 5 was added. The comparative reaction buffer 1 was added so that the reaction solution had a salt concentration of 0.07 M, and a reaction was performed at 25°C for 20 minutes (pH: 7.4). In addition, a similar reaction was performed by using each of the reaction buffers 1 to 5 in place of the comparative reaction buffer 1.

In all Test Examples described herein, the salt concentration of the reaction solution using each reaction buffer is as shown below.

| Reaction buffer Salt concentration of reaction solution | |
|---|---|
| Comparative reaction buffer 1 | 0.07 M |
| Reaction buffer 1 | 0.15 M |
| Reaction buffer 2 | 0.2 M |
| Reaction buffer 3 | 0.25 M |
| Reaction buffer 4 | 0.3 M |
| Reaction buffer 5 | 0.35 M |

Next, the negative control particles after the reaction were washed with a washing buffer (TBS containing 0.1% by mass of Tween 20. The same applies hereinafter.), and 50 µL of alkaline phosphatase (hereinafter abbreviated as ALP) labeled anti CD81 antibody (0.1 µg/mL) diluted with TBS containing 1% by mass of BSA was added, followed by a reaction at 25°C for 20 minutes. After washing with the same washing buffer as above, 50 µL of a luminescent substrate (Class III Series Lumipulse substrate solution, manufactured by Fujirebio Inc.) was added, and after 5 minutes, a luminescence intensity was measured by using a luminescence measuring device (GloMax, manufactured by Promega). The results are shown in FIG. 1-1 and FIG. 1-2.

As shown in FIG. 1-1 and FIG. 1-2, when the salt concentration of the reaction solution was 0.07 M, a background signal attributed to non-specific adsorption of a test sample component was detected, but when the salt concentration of the reaction solution was from 0.15 M to 0.35 M, the non-specific adsorption was reduced and the background signal was reduced.

### (Test Example 2-1)

Reactivity in the case of using the comparative reaction buffer 1 or any one of the reaction buffers 1 to 5 was evaluated by: mixing the anti CD9 antibody bound magnetic particles, a clinical specimen containing an exosome, and the comparative reaction buffer 1 or any one of the reaction buffers 1 to 5; washing the resultant; and then detecting the captured exosome by ELISA in the same manner as in Test Example 1.

To each of 2 mL Protein LoBind tubes (#0030108132, manufactured by Eppendorf), 0.1 mg of the anti CD9 antibody bound magnetic particles were added, and to each of the tubes, any one of the test samples 6 to 8 was added (test sample 6: 0.1 mL, test sample 7: 0.1 mL, test sample 8: 0.1 mL). The comparative reaction buffer 1 was added so that the reaction solution had a salt concentration of 0.07 M, and a reaction was performed at 25°C for 5 hours (pH: 7.4). In addition, a similar reaction was performed by using each of the reaction buffers 1 to 5 in place of the comparative reaction buffer 1.

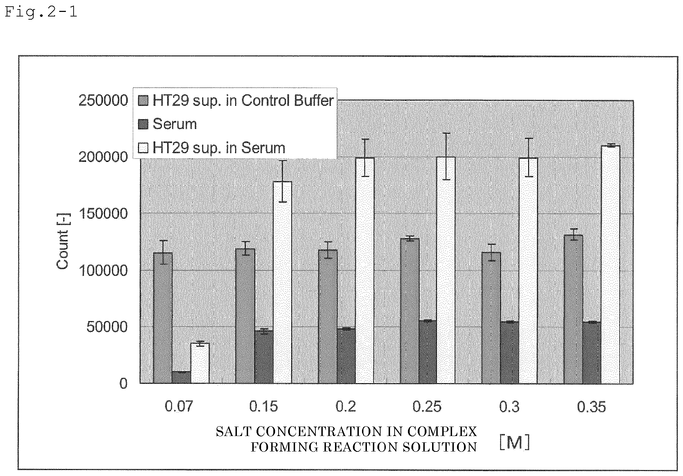

Next, the resultant was washed with 0.5 mL of Washing/Dilution Buffer (manufactured by JSR Life Sciences Corporation), and then dispensed into a 96 well white plate (#23711007, manufactured by Corning Incorporated) at 0.0125 mg/well. In the same manner as in Test Example 1, washing with a washing buffer, a reaction with ALP labeled anti CD81 antibody, and washing with a washing buffer were performed, and then a luminescence intensity was measured. The results are shown in FIG. 2-1.

As shown in FIG. 2-1, it was found that when the salt concentration of the reaction solution was from 0.15 M to 0.35 M, the reactivity of the capturing reaction in blood serum was enhanced.

### (Test Example 2-2)

The same test as that of Test Example 2-1 was performed except that the reaction conditions of the capturing reaction were changed from 25°C and 20 minutes to 4°C and 5 hours.

To each of 2 mL Protein LoBind tubes (#0030108132, manufactured by Eppendorf), 0.1 mg of the anti CD9 antibody bound magnetic particles were added, and to each of the tubes, any one of the test samples 6 to 8 was added at 0.1 mL. The comparative reaction buffer 1 was added so that the reaction solution had a salt concentration of 0.07 M, and a reaction was performed at 4°C for 5 hours (pH: 7.4). In addition, a similar reaction was performed by using the reaction buffer 3 in place of the comparative reaction buffer 1.

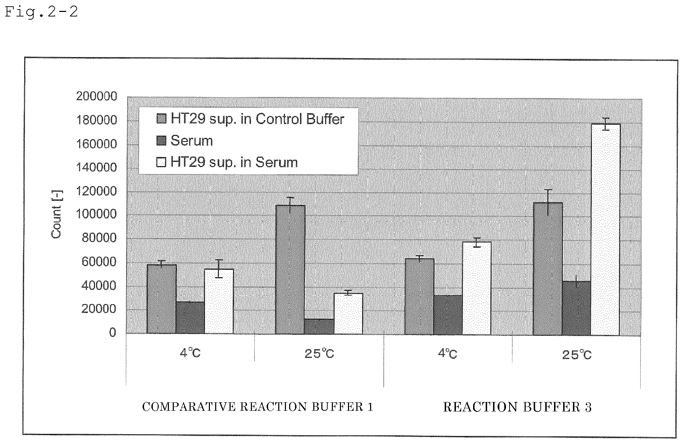

Next, the resultant was washed with 0.5 mL of a washing buffer, and then dispensed into a 96 well white plate (#23711007, manufactured by Corning Incorporated) at 0.0125 mg/well. In the same manner as in Test Example 1, washing with a washing buffer, a reaction with ALP labeled anti CD81 antibody, and washing with a washing buffer were performed, and then a luminescence intensity was measured. The results are shown in FIG. 2-2. In FIG. 2-2, the results of the test performed at a reaction temperature of 25°C are also shown.

It was found from the results of Test Example 2-2 (FIG. 2-2) that, when the salt concentration of the reaction solution was 0.25 M, the reactivity of the capturing reaction in blood serum was satisfactory irrespective of whether the reaction temperature was 4°C or 25°C.

### (Test Example 3)

The non-specific adsorption reducing action of the reaction buffer 3 was investigated for different test samples.

To each of 2 mL Protein LoBind tubes (#0030108132, manufactured by Eppendorf), 0.1 mg of the negative control particles were added, and to each of the tubes, any one of the test samples 9 to 17 was added at 0.1 mL. The comparative reaction buffer 1 was added so that the reaction solution had a salt concentration of 0.07 M, and a reaction was performed at 25°C for 5 hours (pH: 7.4). In addition, a similar reaction was performed by using an equal amount of the reaction buffer 3 in place of the comparative reaction buffer 1.

Next, the resultant was washed with 0.5 mL of a washing buffer, and then dispensed into a 96 well white plate (#23711007, manufactured by Corning Incorporated) at 0.0125 mg/well. In the same manner as in Test Example 1, washing with a washing buffer, a reaction with ALP labeled anti CD81 antibody, and washing with a washing buffer were performed, and then a luminescence intensity was measured.

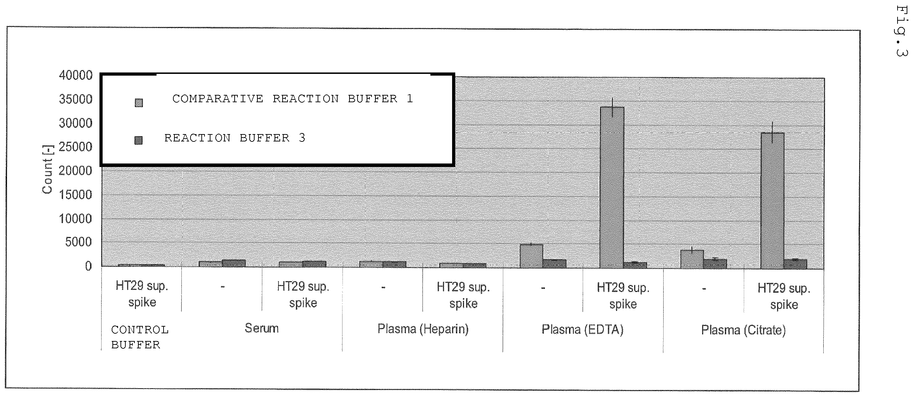

As shown in FIG. 3, when the salt concentration of the reaction solution was 0.25 M, the background signal was small irrespective of the kind of the test sample.

### (Test Example 4)

The reactivity enhancing action of the reaction buffer 3 was investigated for different test samples. A specific procedure is described below.

To each of 2 mL Protein LoBind tubes (#0030108132, manufactured by Eppendorf), 0.1 mg of the anti CD9 antibody bound magnetic particles were added, and to each of the tubes, any one of the test samples 9 to 13 was added at 0.1 mL. The comparative reaction buffer 1 was added so that the reaction solution had a salt concentration of 0.07 M, and a reaction was performed at 25°C for 5 hours (pH: 7.4). In addition, a similar reaction was performed by using the reaction buffer 3 in place of the comparative reaction buffer 1.

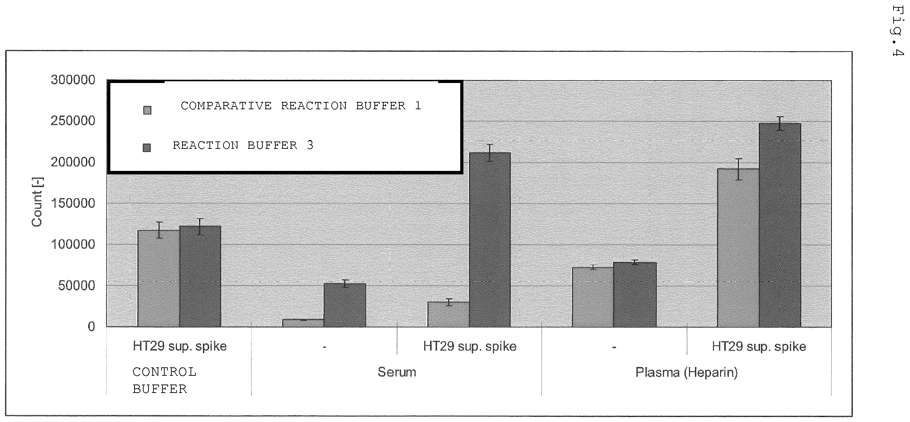

Next, the resultant was washed with 0.5 mL of a washing buffer, and then dispensed into a 96 well white plate (#23711007, manufactured by Corning Incorporated) at 0.0125 mg/well. In the same manner as in Test Example 1, washing with a washing buffer, a reaction with ALP labeled anti CD81 antibody, and washing with a washing buffer were performed, and then a luminescence intensity was measured. The results are shown in FIG. 4.

As shown in FIG. 4, when the salt concentration was 0.25 M, the reactivity of the capturing reaction was high irrespective of the kind of the test sample.

### (Test Example 5)

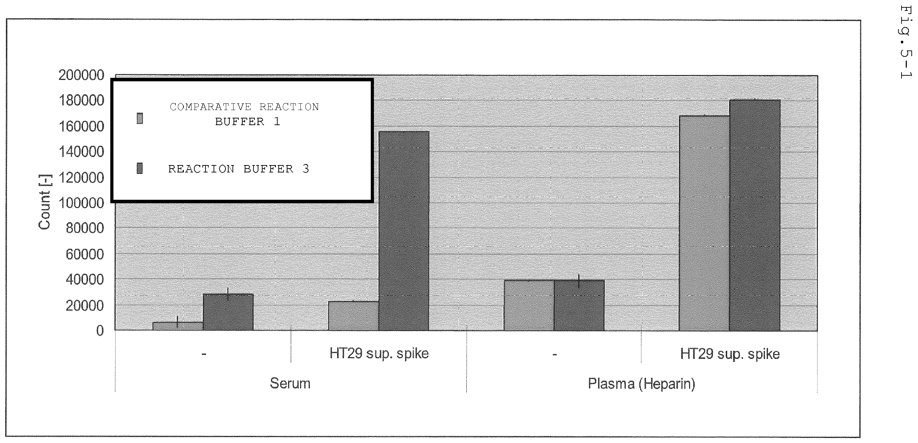

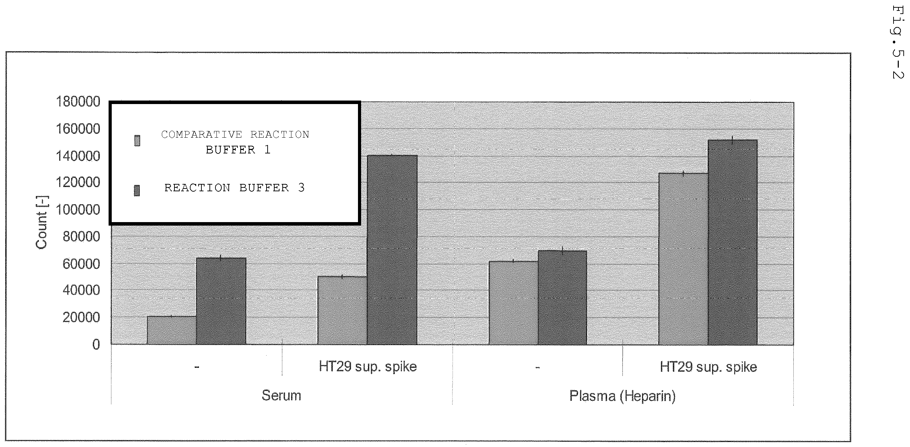

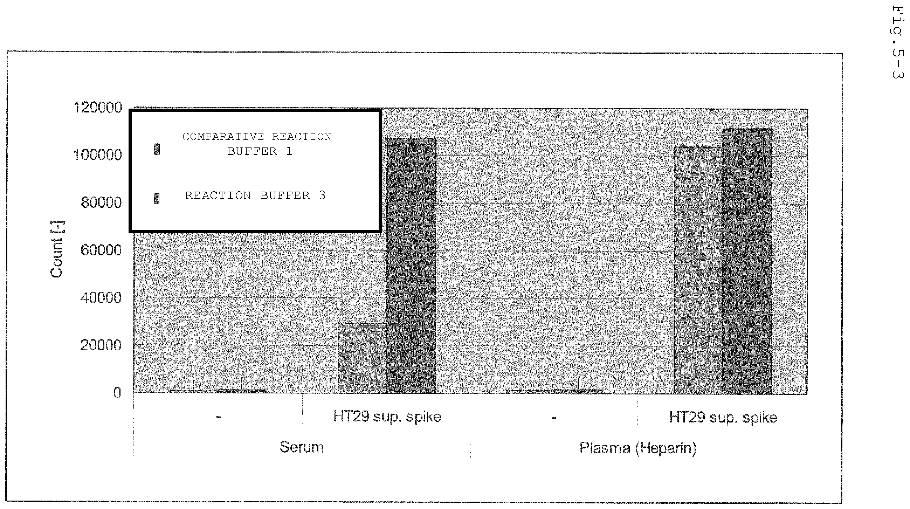

The reactivity enhancing action of the reaction buffer 3 was investigated in the same manner as in Test Example 4 except that: the anti CD9 antibody bound magnetic particles were changed to the anti CD63 antibody bound magnetic particles, the anti CD81 antibody bound magnetic particles, or the anti EpCAM antibody bound magnetic particles; and the test samples 10 to 13 were used as the test samples. The results are shown in FIG. 5-1 (anti CD63 antibody bound magnetic particles), FIG. 5-2 (anti CD81 antibody bound magnetic particles), and FIG. 5-3 (anti EpCAM antibody bound magnetic particles).

### (Test Example 6 - Detection of Nucleic Acid in Vesicle)

Twelve 2 mL Protein LoBind tubes (#0030108132, manufactured by Eppendorf) were prepared and numbered as tubes 1 to 12. 0.1 mg of the anti CD9 antibody bound magnetic particles were added to each of the tubes 1 to 4, 0.5 mg of the anti CD9 antibody bound magnetic particles were added to each of the tubes 5 to 8, and 1 mg of the anti CD9 antibody bound magnetic particles were added to each of the tubes 9 to 12.

Subsequently, to each of the tubes 1 to 12, the reaction buffer 3 was added so that the reaction solution had a salt concentration of 0.25 M, and 1 mL of the test sample 10 was further added. A reaction was performed at 25°C for 1 hour in each of the tubes 1, 5, and 9, at 25°C for 5 hours in each of the tubes 2, 6, and 10, at 25°C for 24 hours in each of the tubes 3, 7, and 11, or at 4°C for 24 hours in each of the tubes 4, 8, and 12.

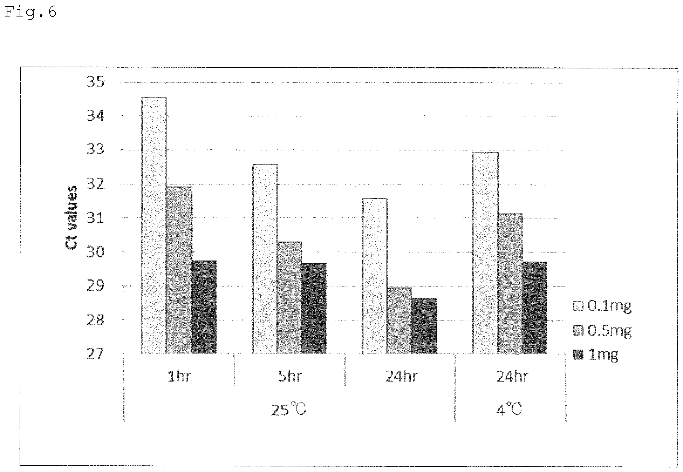

Next, the particles after the reaction in each tube were washed 3 times with 0.5 mL of a washing buffer, and 0.5 mL of the resultant suspension was dispensed into a fresh 2 mL Protein LoBind tube (#0030108132, manufactured by Eppendorf) different from those described above. The antibody bound magnetic particles were collected by magnetic collecting through use of a magnetic collection rack, and then 0.4 mL of the supernatant was removed. From 0.1 mL of the remaining antibody bound particle liquid, microRNA was recovered and quantified. The results are shown in FIG. 6.

The recovery of microRNA was performed using ExoCap™ Nucleic Acid Elution Buffer (#MEX-E, manufactured by MBL). In addition, the quantification of microRNA (miR-21) was performed using TaqMan MicroRNA Reverse Transcription Kit (#4366597, manufactured by Life Technologies Corporation), TaqMan MicroRNA Assays (#186948384, manufactured by Life Technologies Corporation), and TaqMan Universal Master Mix II, no UNG (#4440040, manufactured by Life Technologies Corporation), and the operation was performed in accordance with the recommended protocol.

As shown in FIG. 6, the nucleic acid in the vesicle derived from healthy human blood serum was able to be detected by performing the reaction under the environment in which the reaction solution had a salt concentration of 0.25 M. Further, the amount of the nucleic acid detected increased in a manner dependent on the amount of the antibody bound particles, temperature, and time.

### (Test Example 7 - Detection of Nucleic Acid in Vesicle)

Eight 2 mL Protein LoBind tubes (#0030108132, manufactured by Eppendorf) were prepared and numbered as tubes 13 to 20. 1 mg of the anti CD9 antibody bound magnetic particles were added to each of the tubes 13 and 14, 1 mg of the anti CD63 antibody bound magnetic particles were added to each of the tubes 15 and 16, 1 mg of the anti CD81 antibody bound magnetic particles were added to each of the tubes 17 and 18, and 1 mg of the composite antibody bound magnetic particles (obtained by mixing the anti CD9 antibody bound magnetic particles, the anti CD63 antibody bound magnetic particles, the anti CD81 antibody bound magnetic particles, and the anti EpCAM antibody bound magnetic particles at a ratio of 1:1:1:1) were added to each of the tubes 19 and 20.

Subsequently, to each of the tubes 13 to 20, the reaction buffer 3 was added so that the reaction solution had a salt concentration of 0.25 M. 0.3 mL of the test sample 10 described in Test Example 3 was added to each of the odd-numbered tubes, and 0.3 mL of the test sample 12 described in Test Example 3 was added to each of the even-numbered tubes, followed by a reaction at 25°C for 24 hours.

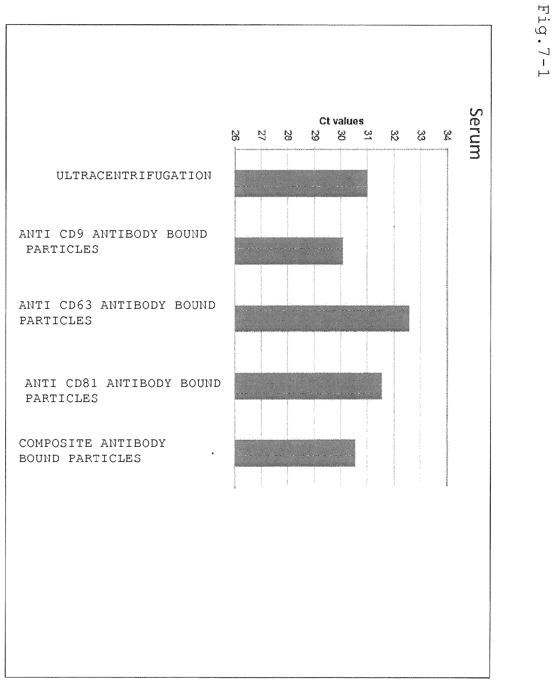

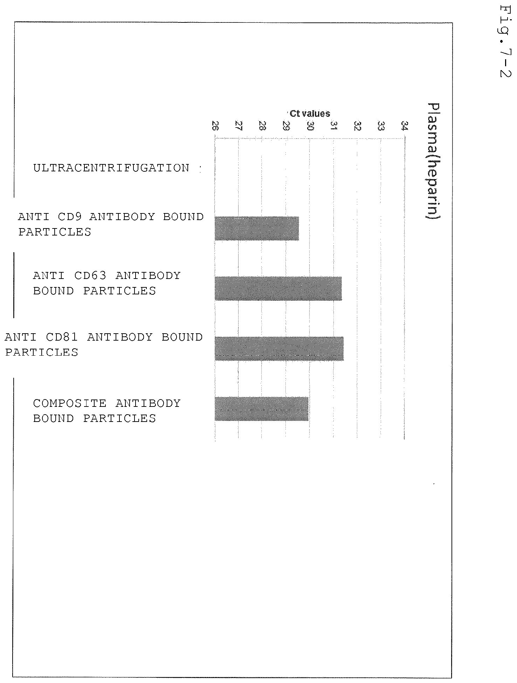

Next, the particles after the reaction in each tube were washed 3 times with 0.5 mL of a washing buffer, and 0.5 mL of the resultant suspension was dispensed into a fresh 2 mL Protein LoBind tube (#0030108132, manufactured by Eppendorf) different from those described above. The antibody bound magnetic particles were collected by magnetic collecting through use of a magnetic collection rack, and then 0.4 mL of the supernatant was removed. From 0.1 mL of the remaining antibody bound particle liquid, microRNA was recovered and quantified. The results are shown in FIG. 7-1 and FIG. 7-2.

The recovery of microRNA was performed using miRNeasy Serum/Plasma Kit (50) (#217184, manufactured by QIAGEN). In addition, the quantification of microRNA (miR-21) was performed in the same manner as in the case of Test Example 6.

In addition, for comparison, the detection of a nucleic acid in a vesicle was performed by using an ultracentrifugation method as a standard method of recovering an exosome. Specifically, 11 mL of each of the test samples 10 and 12 was centrifuged using an ultracentrifuge (Optima XE-90, manufactured by Beckman Coulter) at 100,000×g and 4°C for 70 minutes. The supernatant was removed, and the precipitate was suspended by adding 11 mL of phosphate buffered saline (hereinafter sometimes referred to as "PBS"). The resultant was centrifuged again using an ultracentrifuge (Optima XE-90, manufactured by Beckman Coulter) at 100,000 rpm and 4°C for 70 minutes. The supernatant was removed, and the precipitate was suspended by adding 0.11 mL of PBS. 3 µL of the suspension was used for the recovery and quantification of microRNA. The results are shown in FIG. 7-1 and FIG. 7-2.

The recovery of microRNA and the quantification of microRNA (miR-21) were performed in the same manner as above.

As shown in FIG. 7-1, it was confirmed that the nucleic acid in the vesicle derived from healthy human blood serum was able to be detected at a level comparable to that of the ultracentrifugation method by performing the reaction under the environment in which the salt concentration of the reaction solution was 0.25 M.

As shown in FIG. 7-2, the nucleic acid in the vesicle derived from healthy human heparin blood plasma was not able to be detected when the ultracentrifugation method was used. This is presumably because healthy human heparin blood plasma is considered to generally contain a substance that causes PCR inhibition, and the substance that inhibits PCR remains in a sample recovered by the ultracentrifugation method. Meanwhile, when the reaction was performed under the environment in which the salt concentration of the reaction solution was 0.25 M, the nucleic acid in the vesicle derived from healthy human heparin blood plasma was able to be detected.

As apparent from those results, the nucleic acid in the vesicle was able to be detected irrespective of the kind of the test sample, such as blood serum or blood plasma, by subjecting the vesicle and the particles to the reaction under the environment in which the salt concentration of the reaction solution was 0.25 M.

### (Test Example 8 - Detection of Protein on Vesicle Surface)

Ten 2 mL Protein LoBind tubes (#0030108132, manufactured by Eppendorf) were prepared and numbered as tubes 21 to 30. To each of the tubes, 0.2 mg of magnetic particles were added as shown below.

| Tube numberMagnetic particles | |
|---|---|
| 21 and 22 | Anti CD9 antibody bound magnetic particles |
| 23 and 24 | Anti CD63 antibody bound magnetic particles |
| 25 and 26 | Anti CD81 antibody bound magnetic particles |
| 27 and 28 | Anti EpCAM antibody bound magnetic particles |
| 29 and 30 | Composite antibody bound magnetic particles |

Subsequently, to each of the tubes 21 to 30, the reaction buffer 3 was added so that the reaction solution had a salt concentration of 0.25 M. 1.0 mL of the test sample 10 described in Test Example 3 was added to each of the odd-numbered tubes, and 1.0 mL of the test sample 12 described in Test Example 3 was added to each of the even-numbered tubes, followed by a reaction at 25°C for 3 hours.

Next, the particles after the reaction in each tube were washed 3 times with 0.5 mL of a washing buffer, and 0.5 mL of the resultant suspension was dispensed into a fresh 2 mL Protein LoBind tube (#0030108132, manufactured by Eppendorf) different from those described above.

Subsequently, after discarding the washing solution by magnetic collecting, the antibody bound magnetic particles were suspended in 20 µL of a 1× sample buffer and allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer.

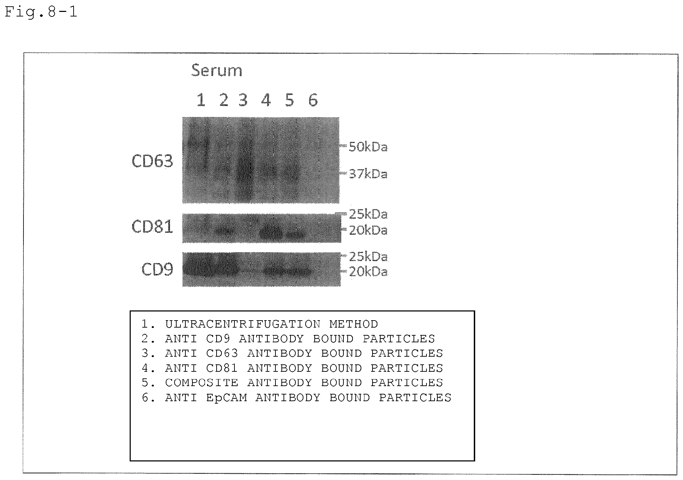

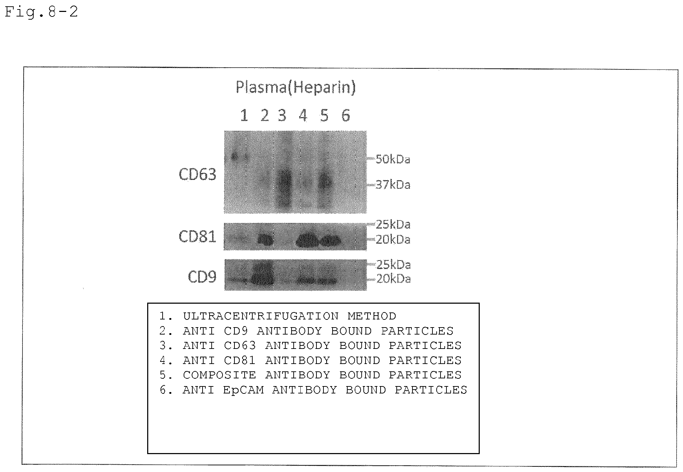

In the same manner as in Test Example 7, a reaction with a primary antibody, and washing with a washing buffer were performed, and the resultant was subjected to a reaction with a luminescent substrate. A western blot image was observed with a luminescence measuring device. The results are shown in FIG. 8-1 and FIG. 8-2.

In addition, for comparison, the detection of a protein on a surface of a vesicle was performed by using an ultracentrifugation method as a standard method of recovering an exosome. Specifically, 11 mL of each of the test samples 10 and 12 was centrifuged using an ultracentrifuge (Optima XE-90, manufactured by Beckman Coulter) at 100,000×g and 4°C for 70 minutes. The supernatant was removed, and the precipitate was suspended by adding 11 mL of PBS. The resultant was centrifuged again using an ultracentrifuge (Optima XE-90, manufactured by Beckman Coulter) at 100,000×g and 4°C for 70 minutes. The supernatant was removed, and the precipitate was suspended by adding 0.11 mL of PBS. To 10 µL of the suspension, 5 µL of a 4× sample buffer and 5 µL of PBS were added, and the resultant was allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer.

In the same manner as in Test Example 8, a reaction with a primary antibody, and washing with a washing buffer were performed, and the resultant was subjected to a reaction with a luminescent substrate. A western blot image was observed with a luminescence measuring device. The results are shown in FIG. 8-1 and FIG. 8-2.

As shown in FIG. 8-1 and FIG. 8-2, it was confirmed that the protein on the surface of the vesicle derived from each of healthy human blood serum and healthy human heparin blood plasma was able to be detected at a level comparable to that of the ultracentrifugation method by subjecting the vesicle and the particles to the reaction under the environment in which the salt concentration of the reaction solution was 0.25 M. In addition, when the anti EpCAM antibody bound magnetic particles were used, no vesicle surface protein was detected. EpCAM protein is hardly present in blood of a healthy person and its blood concentration is considered to increase owing to a disease, such as cancer. A non-specific reaction was able to be suppressed by subjecting the vesicle and the particles to the reaction under the environment in which the salt concentration of the reaction solution was 0.25 M.

### (Test Example 9-1 - Detection of Protein Maintaining Three-dimensional Structure of Native Form on Vesicle Surface (1))

Eight 2 mL Protein LoBind tubes (#0030108132, manufactured by Eppendorf) were prepared and numbered as tubes 31 to 38. To each of the tubes, 0.1 mg of magnetic particles were added as shown below.

| Tube numberMagnetic particles | |
|---|---|
| 31 and 32 | Anti CD9 antibody bound magnetic particles |
| 33 and 34 | Anti CD63 antibody bound magnetic particles |
| 35 and 36 | Anti CD81 antibody bound magnetic particles |
| 37 and 38 | Anti EpCAM antibody bound magnetic particles |

The reaction buffer 3 was added to each of the tubes 31 to 38 so that the reaction solution had a salt concentration of 0.25 M.

Subsequently, 0.3 mL of the test sample 10 described in Test Example 3 was added to each of the tubes 31, 33, 35, and 37, followed by a reaction at 25°C overnight. In addition, 0.3 mL of the test sample 12 described in Test Example 3 was added to each of the tubes 32, 34, 36, and 38, followed by a reaction at 25°C overnight.

Next, the particles after the reaction in each tube were washed 2 times with 0.25 mL of a washing buffer, and after magnetic collecting, the supernatant was removed, followed by suspension in 1 mL of phosphate buffered saline.

40 fresh 2 mL Protein LoBind tubes (#0030108132, manufactured by Eppendorf) different from those described above were prepared and numbered as tubes 39 to 78. 0.1 mL of each of the suspensions in the tubes 31 to 38 was dispensed as described below.

The suspension in the tube 31 was dispensed into each of the tubes 39 to 43.

The suspension in the tube 32 was dispensed into each of the tubes 44 to 48.

The suspension in the tube 33 was dispensed into each of the tubes 49 to 53.

The suspension in the tube 34 was dispensed into each of the tubes 54 to 58.

The suspension in the tube 35 was dispensed into each of the tubes 59 to 63.

The suspension in the tube 36 was dispensed into each of the tubes 64 to 68.

The suspension in the tube 37 was dispensed into each of the tubes 69 to 73.

The suspension in the tube 38 was dispensed into each of the tubes 74 to 78.

Subsequently, to each of the tubes, 5 µL of an antibody was added as shown below.

| Tube number | Antibody |
|---|---|
| 39, 44, 49, 54, 59, 64, 69, and 74 IgG | PE anti mouse |
| 40, 45, 50, 55, 60, 65, 70, and 75 | PE anti CD9 |
| 41, 46, 51, 56, 61, 66, 71, and 76 | PE anti CD63 |
| 42, 47, 52, 57, 62, 67, 72, and 77 | PE anti CD81 |
| 43, 48, 53, 58, 63, 68, 73, and 78 | PE anti EpCAM |

PE anti mouse IgG: phycoerythrin (hereinafter abbreviated as PE) labeled anti mouse IgG antibody (manufactured by SONY)
PE anti CD9: PE labeled anti CD9 antibody (manufactured by SONY)
PE anti CD63: PE labeled anti CD63 antibody (manufactured by SONY)
PE anti CD81: PE labeled anti CD81 antibody (manufactured by SONY)
PE anti EpCAM: PE labeled anti EpCAM antibody (manufactured by SONY)

After that, the tubes 39 to 78 were suspended under the conditions of 25°C and 1,000 rpm for 1 hour.

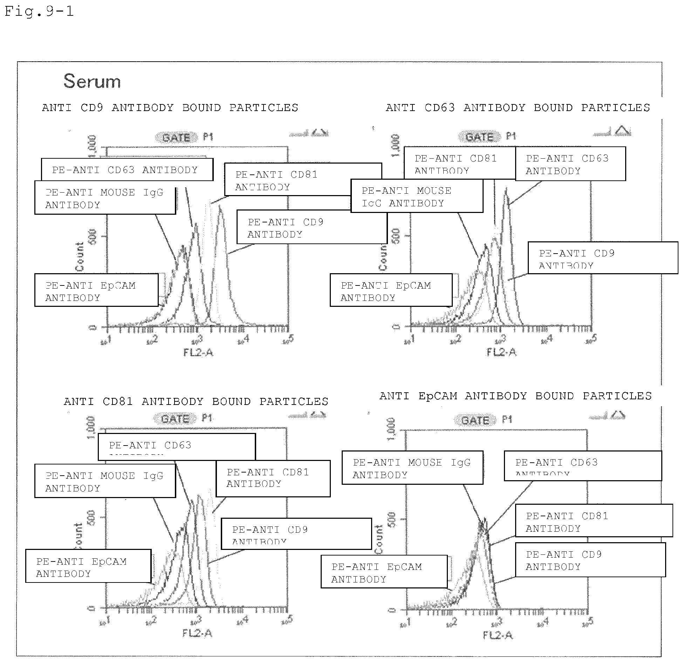

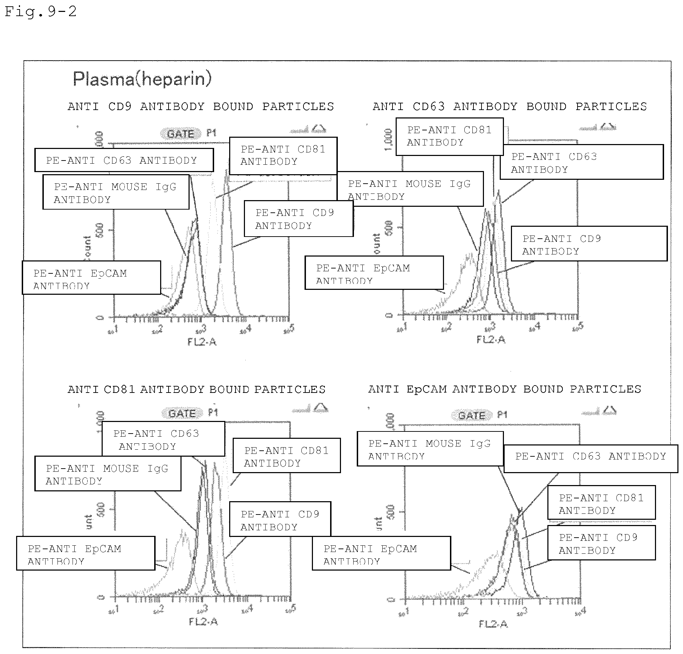

Next, the particles after the reaction in each tube were washed 2 times with 0.5 mL of phosphate buffered saline, and then a fluorescence signal of each sample was observed using a flow cytometer (BD Accuri C6, manufactured by BD). The results are shown in FIG. 9-1 and FIG. 9-2.

As shown in FIG. 9-1 and FIG. 9-2, CD9, CD63, and CD81 on the surface of the vesicle derived from each of healthy human blood serum and healthy human heparin blood plasma were able to be detected by subjecting the vesicle and the particles to the reaction under the environment in which the salt concentration of the reaction solution was 0.25 M.

In addition, when the anti CD9 antibody bound magnetic particles, the anti CD63 antibody bound magnetic particles, or the anti CD81 antibody bound magnetic particles were used, the peak of EpCAM, which was considered to be expressed by oncogenesis, detected with the anti EpCAM antibody did not show a peak shift as compared to that in the case of the anti mouse IgG antibody serving as a control. It is considered that EpCAM is not detected from a test sample from a healthy person.

Further, when the anti EpCAM antibody bound magnetic particles were used as a solid phase carrier, the peak detected with the anti mouse IgG antibody did not differ very much from the peak detected with the anti CD9 antibody, the anti CD63 antibody, the anti CD81 antibody, or the anti EpCAM antibody. That is, when the anti EpCAM antibody bound magnetic particles were used as a solid phase carrier, an exosome was not obtained from a test sample from a healthy person. It is considered that non-specific adsorption is low.

In addition, the samples used for the analysis were not treated with heat, a chemical, or the like changing the three-dimensional structure of a protein, and hence it is considered that CD9, CD63, and CD81 of native forms present on the vesicle surface were able to be detected.

(Test Example 9-2 - Detection of Protein Maintaining Three-dimensional Structure of Native Form on Vesicle Surface (2))

Prior to the test, test samples 18 and 19 described below were prepared.

Eight 2 mL Protein LoBind tubes (#0030108132, manufactured by Eppendorf) were prepared and numbered as tubes 79 to 86. 0.1 mg of the anti CD9 antibody bound magnetic particles were added to each of the tubes 79 and 80, 0.1 mg of the anti CD63 antibody bound magnetic particles were added to each of the tubes 81 and 82, 0.1 mg of the anti CD81 antibody bound magnetic particles were added to each of the tubes 83 and 84, and 0.1 mg of the anti EpCAM antibody bound magnetic particles were added to each of the tubes 85 and 86. The reaction buffer 3 was added to each of the tubes 79 to 86 so that the reaction solution had a salt concentration of 0.25 M.

Subsequently, 0.3 mL of the test sample 18 was added to each of the tubes 79, 81, 83, and 85, followed by a reaction at 25°C overnight. In addition, 0.3 mL of the test sample 19 was added to each of the tubes 80, 82, 84, and 86, followed by a reaction at 25°C overnight.

Next, the particles after the reaction in each tube were washed 2 times with 0.25 mL of a washing buffer, and after magnetic collecting, the supernatant was removed, followed by suspension in 1 mL of phosphate buffered saline.

40 fresh 2 mL Protein LoBind tubes (#0030108132, manufactured by Eppendorf) different from those described above were prepared and numbered as tubes 87 to 126. 0.1 mL of each of the suspensions in the tubes 79 to 86 was dispensed as described below.

The suspension in the tube 79 was dispensed into each of the tubes 87 to 91.

The suspension in the tube 80 was dispensed into each of the tubes 92 to 96.

The suspension in the tube 81 was dispensed into each of the tubes 97 to 101.

The suspension in the tube 82 was dispensed into each of the tubes 102 to 106.

The suspension in the tube 83 was dispensed into each of the tubes 107 to 111.

The suspension in the tube 84 was dispensed into each of the tubes 112 to 116.

The suspension in the tube 85 was dispensed into each of the tubes 117 to 121

The suspension in the tube 86 was dispensed into each of the tubes 122 to 126.

Subsequently, to each of the tubes, 5 µL of an antibody was added as shown below.

| Tube number | Antibody |
|---|---|
| 87, 92, 97, 102, 107, 112, 117, and 122 | PE anti mouse IgG |
| 88, 93, 98, 103, 108, 113, 118, and 123 | PE anti CD9 |
| 89, 94, 99, 104, 109, 114, 119, and 124 | PE anti CD63 |
| 90, 95, 100, 105, 110, 115, 120, and 125 | PE anti CD81 |
| 91, 96, 101, 106, 111, 116, 121, and 126 | PE anti EpCAM |

After that, the tubes 87 to 126 were suspended under the conditions of 25°C and 1,000 rpm for 1 hour.

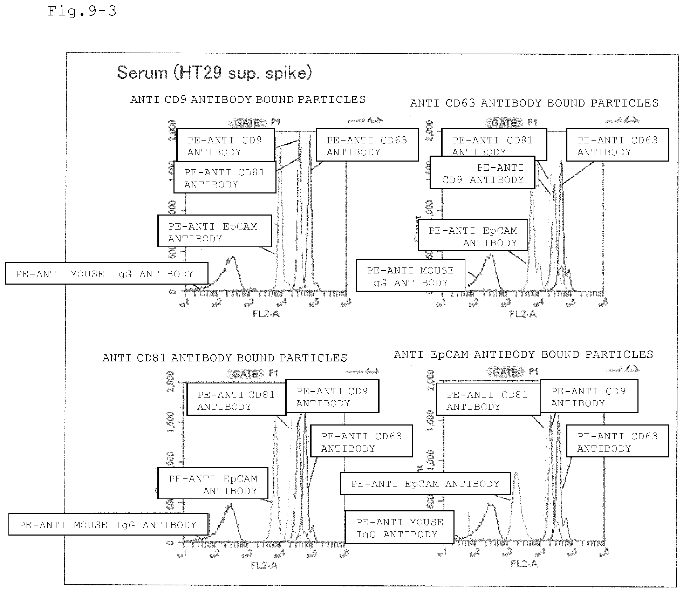

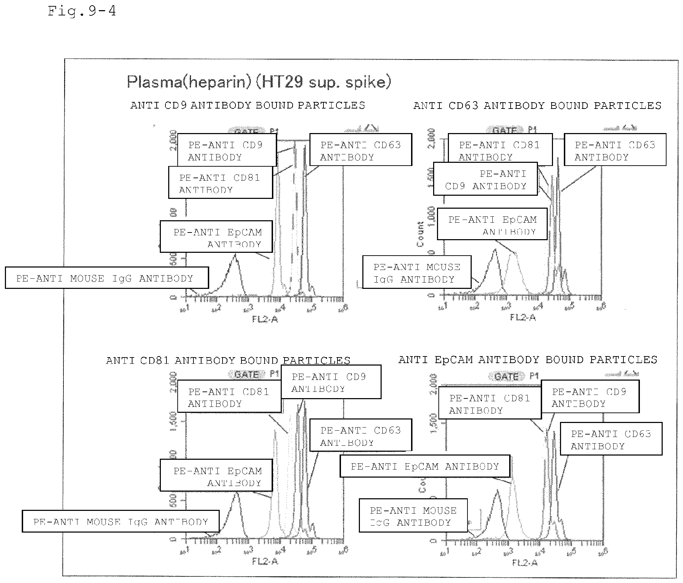

Next, the particles after the reaction in each tube were washed 2 times with 0.5 mL of phosphate buffered saline, and then a fluorescence signal of each sample was observed using a flow cytometer (BD Accuri C6, manufactured by BD). The results are shown in FIG. 9-3 and FIG. 9-4.

As shown in FIG. 9-3 and FIG. 9-4, CD9, CD63, and CD81 present on the surface of the vesicle derived from each of healthy human blood serum, healthy human heparin blood plasma, and human colon cancer HT29 cells were able to be detected by subjecting the vesicle and the particles to the reaction under the environment in which the salt concentration of the reaction solution was 0.25 M.

In addition, the peak of EpCAM, which was considered to be expressed by oncogenesis, detected with the anti EpCAM antibody showed a peak shift as compared to that in the case of the anti mouse IgG antibody serving as a control. This peak shift was not observed in FIG. 9-1 and FIG. 9-2, in which only test samples from healthy persons were used as samples, and hence it is considered that the present invention can be used to determine a disease.

Further, when the anti EpCAM antibody bound magnetic particles were used as a solid phase carrier, the peak detected with the anti mouse IgG antibody did not differ very much from the peak detected with the anti CD9 antibody, the anti CD63 antibody, the anti CD81 antibody, or the anti EpCAM antibody. That is, when the anti EpCAM antibody bound magnetic particles were used as a solid phase carrier, an exosome was not obtained from a test sample from a healthy person. It is considered that non-specific adsorption is low.

In addition, the samples used for the analysis were not treated with heat, a chemical, or the like changing the three-dimensional structure of a protein, and hence it is considered that CD9, CD63, and CD81 of native forms present on the vesicle surface were able to be detected.

### (Test Example 10)

Twenty 2 mL Protein LoBind tubes (#0030108132, manufactured by Eppendorf) were prepared and numbered as tubes 127 to 146. To each of the tubes, 0.2 mg of magnetic particles were added as shown below.

| Tube numberMagnetic particles |
|---|
| 127 to 130 Anti CD9 antibody bound magnetic particles |
| 131 to 134 Anti CD63 antibody bound magnetic particles |
| 135 to 138 Anti CD81 antibody bound magnetic particles |
| 139 to 142 Anti EpCAM antibody bound magnetic particles |
| 143 to 146 Composite antibody bound magnetic particles |

Subsequently, to each of the tubes 127 to 146, the reaction buffer 3 was added so that the reaction solution had a salt concentration of 0.25 M. To each of the tubes, 0.1 mL of a test sample was added as shown below, followed by a reaction at 25°C for 24 hours.

| Tube number | Test sample |
|---|---|
| 127, 131, 135, 139, and 143 | Test sample 10 |
| 128, 132, 136, 140, and 144 | Test sample 18 |
| 129, 133, 137, 141, and 145 | Test sample 12 |
| 130, 134, 138, 142, and 146 | Test sample 19 |

Next, the particles after the reaction in each tube were washed 3 times with 0.5 mL of a washing buffer, and 0.5 mL of the resultant suspension was dispensed into a fresh 2 mL Protein LoBind tube (#0030108132, manufactured by Eppendorf) different from those described above.

Subsequently, after discarding the washing solution by magnetic collecting, the antibody bound magnetic particles were suspended in 20 µL of a 1× sample buffer and allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer.

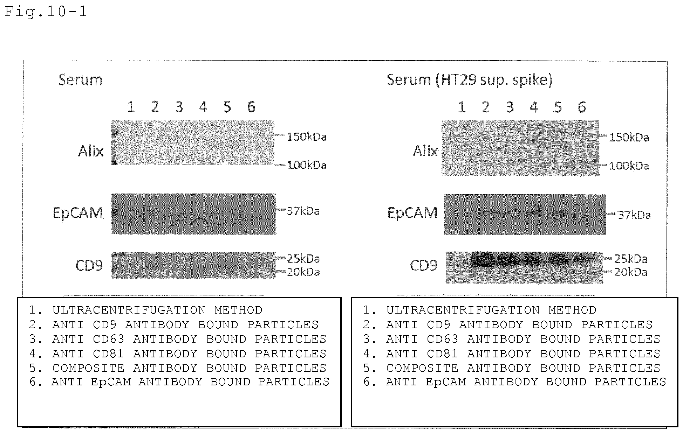

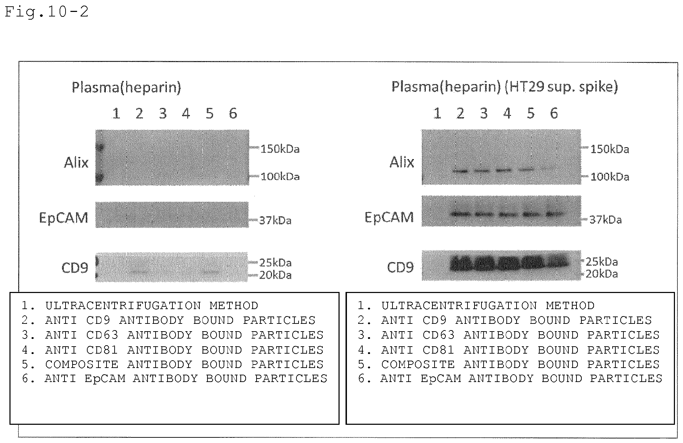

In the same manner as in Test Example 8, a reaction with a primary antibody, and washing with a washing buffer were performed, and the resultant was subjected to a reaction with a luminescent substrate. A western blot image was observed with a luminescence measuring device. The results are shown in FIG. 10-1 and FIG. 10-2.

In addition, for comparison, the detection of a protein on a surface of a vesicle was performed by using an ultracentrifugation method as a standard method of recovering an exosome. Specifically, 11 mL of each of the test samples 10, 12, 18, and 19 was centrifuged using an ultracentrifuge (Optima XE-90, manufactured by Beckman Coulter) at 100, 000×g and 4°C for 70 minutes. The supernatant was removed, and the precipitate was suspended by adding 11 mL of PBS. The resultant was centrifuged again using an ultracentrifuge (Optima XE-90, manufactured by Beckman Coulter) at 100,000×g and 4°C for 70 minutes. The supernatant was removed, and the precipitate was suspended by adding 0.11 mL of PBS. To 1 µL of the suspension, 5 µL of a 4× sample buffer and 14 µL of PBS were added, and the resultant was allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer.

In the same manner as in Test Example 8, a reaction with a primary antibody, and washing with a washing buffer were performed, and the resultant was subjected to a reaction with a luminescent substrate. A western blot image was observed with a luminescence measuring device. The results are shown in FIG. 10-1 and FIG. 10-2.

As shown in FIG. 10-1 and FIG. 10-2, the protein on the surface of the vesicle derived from each of healthy human blood serum, healthy human heparin blood plasma, and human colon cancer HT29 cells was able to be detected at a level comparable to or higher than that of the ultracentrifugation method by subjecting the vesicle and the particles to the reaction under the environment in which the salt concentration of the reaction solution was 0.25 M. In addition, in the case of detection using the anti EpCAM antibody, no band was detected for healthy human blood serum or heparin blood plasma, but when the culture supernatant of the human colon cancer HT29 cells was added thereto, a band was detected. It is considered that the present invention can be used to determine a disease.

### [Method of Separating Vesicle Having Lipid Bilayer Membrane (First Separation Method)]

A first separation method of the present invention comprises: a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier; and a washing step of washing the complex, in which at least any one of the complex forming step and the washing step is performed in the presence of an inorganic salt or an organic salt at a final concentration of from 0.15 M to 2 M. Herein, the final concentration (end concentration) refers to the concentration of an inorganic salt (generally about 0.14 M), an inorganic salt not including an organic salt, or an organic salt, which is derived from the biological sample, and refers to the end concentration of an inorganic salt or an organic salt in a system artificially caused to be present at the time of the complex forming step or the washing step. The final concentration of the inorganic salt or the organic salt may be adjusted by any method based on a solid material of a powder shape, a particle shape, or the like, or a liquid composition, such as a diluent for specimen. In addition, the final concentration may be measured by using, for example, any of known various measurement methods or measurement devices.

### (Complex Forming Step)

The complex forming step is a step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier. Through the contact, the vesicle is captured by the ligand, and the complex of the vesicle and the solid phase carrier is formed. In a reaction system for the complex forming step, a ligand which recognizes a surface antigen present on a surface of the vesicle having a lipid bilayer membrane other than the ligand bound to the solid phase carrier may be also present.

The biological sample is not particularly limited as long as the biological sample contains a vesicle having a lipid bilayer membrane, and examples thereof include various liquids, such as body fluid, bacterial liquid, cell culture medium, cell culture supernatant, and liquid containing disrupted tissue cells. Of those, body fluid and cell culture supernatant are preferred, and body fluid is more preferred. Examples of the body fluid include components of blood composition, such as whole blood, blood serum, blood plasma, blood components, various blood cells, blood clot, and blood platelet, and also urine, semen, milk, sweat, interstitial fluid, interstitial lymph, bone marrow fluid, tissue fluid, saliva, gastric juice, joint fluid, pleural fluid, bile acid, ascite, and amniotic fluid. Of those, components of blood composition are preferred. In the first separation method of the present invention, even when such clinical specimen is used as the biological sample, non-specific adsorption onto the solid phase carrier is low and the reactivity of a capturing reaction is high. Accordingly, according to the first separation method of the present invention, the vesicle can be separated selectively and efficiently from a wide variety of biological samples. For example, even when blood plasma is used as the biological sample, non-specific adsorption hardly occurs, and even when blood serum is used, the reactivity of the capturing reaction is high.

The components of blood composition may be treated with an anti-clotting agent, such as citric acid, heparin, or EDTA.

The biological sample may be used as a pre-treated sample after being, for example, added in advance to a buffer composition containing an inorganic salt or an organic salt in such an amount as to allow a final concentration of from 0.15 M to 2 M. Alternatively, a sample collected from a living organism may be used as it is. That is, according to the first separation method of the present invention, a simple and selective separation can be made without performing a pre-treatment based on an isolation method or the like using PEG precipitation, an ultracentrifuge, or the like.

In addition, although examples of the vesicle having a lipid bilayer membrane include a cell and a vesicle such as an exosome which is discharged extracellularly from a cell, the first separation method of the present invention is more preferably used for a case in which the vesicle is an exosome.

In addition, the surface antigen present on a surface of the vesicle is not particularly limited as long as the surface antigen is a substance present on a vesicle surface and having antigenicity. A surface antigen of an exosome is exemplified by: tetraspanins, such as CD9, CD63, and CD81; proteins related to antigen-presentation, such as MHCI and MHCII; adhesion molecules, such as integrin, ICAM-1, and EpCAM; cytokines, such as EGFRvIII and TGF-β/cytokine receptors, and enzymes. Of those, an antigen protein present on an exosome surface is preferred.

In addition, the solid phase carrier to be used for the first separation method of the present invention is not particularly limited as long as the solid phase carrier has bound thereto a ligand which recognizes a surface antigen present on a surface of a vesicle.

The ligand is preferably an antibody which recognizes a surface antigen present on a surface of a vesicle, more preferably an antibody which recognizes a surface antigen present on a surface of an exosome. In addition, the ligand may be a monoclonal antibody or a polyclonal antibody, and is preferably a monoclonal antibody.

The monoclonal antibody is not particularly limited, and may be an antibody produced in accordance with a known method, for example, a method described in K. Watanabe et al. , Vasohibin as an endothelium-derived negative feedback regulator of angiogenesis, J. Clin. Invest. 114 (2004), 898-907. In addition, a monoclonal antibody which recognizes any of tetraspanins, such as surface antigens CD9, CD63, and CD81 of an exosome, may be prepared with reference to WO 2013/099925 A1 or the like.

Examples of the class of the antibody include IgG and IgM. Of those, IgG is preferred. In addition, a fragment obtained by decomposing any such antibody into a small molecule may also be used. Examples thereof include F(ab')2, Fab', and Fab.

A material of the solid phase carrier for binding the above-mentioned ligand is exemplified by: a high molecular compound, such as polystyrenes, polyethylenes, polypropylenes, polyesters, poly(meth)acrylonitriles, a styrene-butadiene copolymer, poly(meth)acrylic acid esters, a fluororesin, cross-linked dextran, or polysaccharides; glass; a metal; a magnetic material; a resin composition containing a magnetic material; and a combination thereof.

In addition, the shape of the solid phase carrier is not particularly limited, and examples thereof include a tray, a globule, a particle, a fiber, a rod, a dish, a container, a cell, a micro plate, and a test tube.

In the present invention, from the viewpoint of easiness in solid-liquid separation or washing, magnetic particles are preferred.

Examples of the magnetic particles include: a metal, such as ferric oxide (Fe₃O₄), maghemite (γ-Fe₂O₃), various ferrites, iron, manganese, nickel, cobalt, or chromium; magnetic fine particles formed of an alloy of, for example, cobalt, nickel, or manganese; and magnetic particles containing those magnetic materials in a resin. Examples of the resin include a hydrophobic polymer and a hydrophilic polymer.

Of those, magnetic particles each containing a magnetic material in a resin are preferred, and magnetic particles each obtained by forming a polymer layer on a surface of a parent particle containing superparamagnetic fine particles are more preferred. Examples thereof include magnetic particles described in JP-A-2008-32411 each obtained by forming a hydrophobic first polymer layer on a surface of a parent particle containing superparamagnetic fine particles, forming, on the first polymer layer, a second polymer layer having a glycidyl group at least on a surface thereof, and introducing a polar group therein by chemical modification of the glycidyl group.

Typical examples of the superparamagnetic fine particles include fine particles of iron oxide each having a particle diameter of 20 nm or less (preferably a particle diameter of from 5 nm to 20 nm), and examples thereof include ferrite represented by XFe₂O₄ (X represents Mn, Co, Ni, Mg, Cu, Li_{0.5}Fe_{0.5}, or the like), magnetite represented by Fe₃O₄, and γ-Fe₂O₃. From the viewpoint of having strong saturation magnetization and little residual magnetization, the superparamagnetic fine particles each preferably contain any one of γ-Fe₂O₃ and Fe₃O₄.

In addition, monomers for forming the hydrophobic first polymer layer are roughly classified into a monofunctional monomer and a crosslinkable monomer.

Examples of the monofunctional monomer include: an aromatic vinyl monomer, such as styrene, α-methyl styrene, or halogenated styrene; and an ethylenically unsaturated carboxylic acid alkyl ester monomer, such as methyl acrylate, methyl methacrylate, ethyl acrylate, ethyl methacrylate, stearyl acrylate, stearyl methacrylate, cyclohexyl acrylate, cyclohexyl methacrylate, isobornyl acrylate, or isobornyl methacrylate. In addition, examples of the crosslinkable monomer include: a polyfunctional (meth)acrylate, such as ethylene glycol diacrylate, ethylene glycol dimethacrylate, trimethylol propane triacrylate, trimethylol propane trimethacrylate, pentaerythritol triacrylate, pentaerythritol trimethacrylate, dipentaerythritol hexaacrylate, or dipentaerythritol hexamethacrylate; a conjugated diolefin, such as butadiene or isoprene; and divinyl benzene, diallyl phthalate, allyl acrylate, and allyl methacrylate.

In addition, monomers for forming the second polymer layer are mainly intended to introduce a functional group to a particle surface, and include a monomer containing a glycidyl group. The content of the monomer containing a glycidyl group is preferably 20% by mass or more in all the monomers for forming the second polymer layer. Examples of the copolymerizable monomer containing a glycidyl group include glycidyl acrylate, glycidyl methacrylate, and allyl glycidyl ether.

The polar group to be introduced by chemical modification of the glycidyl group in the second polymer layer is preferably a functional group which can react with a ligand, more preferably a group containing one or more of at least one kind of atom selected from the group consisting of an oxygen atom, a nitrogen atom, and a sulfur atom. Of those, an amino group, an aldehyde group, a carboxy group, and an active ester group are more preferred. Particularly when the second polymer layer of each of the magnetic particles contains the polar group and a 2, 3-dihydroxypropyl group, a good binding property with a ligand is obtained.

In addition, the solid phase carrier, such as magnetic particles, may have immobilized on a surface thereof a substance having a binding site for biotin or an analog thereof. Examples of the substance having a binding site for biotin or an analog thereof include one kind or two or more kinds of compounds selected from avidin, streptavidin, tamavidin, and derivatives thereof. Further, in this case, the solid phase carrier may have bound thereto an antibody or the like bound with biotin or an analog thereof, via the substance having a binding site for biotin or an analog thereof. The immobilization of the substance having a binding site for biotin or an analog thereof onto the surface of the solid phase carrier may be performed by a known method, such as a method described in JP-B-4716034.

As a method of binding the ligand to the solid phase carrier, for example, a physical adsorption method and a chemical binding method, such as a covalent bond method or an ionic bond method, is used. Examples of the physical adsorption method include a method involving directly immobilizing the ligand onto the solid phase carrier and a method involving chemically binding the ligand to another protein, such as albumin, followed by immobilization based on adsorption. Examples of the chemical binding method include a method involving directly binding the ligand onto the solid phase carrier by using a functional group introduced to the surface of the solid phase carrier, in which the functional group is capable of reacting with the ligand, a method involving carrying out binding after introduction of a spacer molecule (for example, carbodiimide compound) between the solid phase carrier and the ligand by chemical binding, and a method involving binding the ligand to another protein, such as albumin, followed by chemical binding of the protein to the solid phase carrier.

In addition, a commercially available product may be used as the solid phase carrier to which a ligand which recognizes a surface antigen present on a surface of the vesicle is bound. Examples thereof include Exosome-Human CD9 Isolation Reagent (from cell culture) and Exosome-Human CD63 Isolation/Detection Reagent (from cell culture media) (each of which is manufactured by Life Technologies Corporation), and CD9 Exo-Flow capture kit, CD63 Exo-Flow capture kit, and CD81 Exo-Flow capture kit (all of which are manufactured by SBI).

In addition, the used amount of the solid phase carrier to which the ligand is bound is preferably from 0.00001 times by mass to 0.1 times by mass, more preferably from 0.0001 times by mass to 0.01 times by mass, with respect to the biological sample.

The complex forming step may be performed by using, as necessary: a surfactant; a protein, such as albumin; a nucleic acid; a saccharide, such as sucrose; or the like in addition to each component described above.

Of those, from the viewpoint of reducing non-specific adsorption and the viewpoint of suppressing damage to the vesicle, a surfactant is preferred, a nonionic surfactant is more preferred, and a polyethylene glycol type nonionic surfactant is even more preferred. In addition, the surfactant is preferably a surfactant not containing an aromatic group in the molecule.

Examples thereof include higher alcohol ethylene oxide adducts, fatty acid ethylene oxide adducts, polyhydric alcohol fatty acid ester ethylene oxide adducts, higher alkylamine ethylene oxide adducts, fatty acid amide ethylene oxide adducts, ethylene oxide adducts of oils and fats, and polyalkylene glycol ethylene oxide adducts. Of those, polyalkylene glycol ethylene oxide adducts are preferred. Specific examples thereof include polyoxyethylene (160) polyoxypropylene (30) glycol and polyoxyethylene (196) polyoxypropylene (67) glycol.

The used amount of the surfactant is preferably from 0.005% (w/v) to 10% (w/v), more preferably from 0.015% (w/v) to 3.5% (w/v), in terms of final concentration.

In addition, the reaction temperature in the complex forming step falls within preferably the range of from 2°C to 42°C, more preferably the range of from 15°C to 40°C, even more preferably the range of from 20°C to 37°C. The complex forming step in the first separation method of the present invention is extremely simple because the reaction proceeds selectively and efficiently even at normal temperature or around normal temperature. In addition, the reaction time is generally from 1 minute to 48 hours, and is preferably from 5 minutes to 24 hours, more preferably from 10 minutes to 10 hours, even more preferably from 10 minutes to 5 hours.

In the complex forming step, the pH in the system is not particularly limited, but the pH preferably falls within the range of from 5 to 10, and the pH more preferably falls within the range of from 6 to 8. In order to maintain a desired pH, a buffer solution is generally used. Examples thereof include a phosphate buffer solution, a tris(hydroxymethyl)aminomethane buffer solution, a HEPES buffer solution, and an MES buffer solution.

### (Washing Step)

The washing step is a step of washing the complex of the vesicle and the solid phase carrier, which has been formed in the complex forming step. According to this step, an unreacted component, an unreacted labeling material, and the like are removed. A system containing the complex of the complex forming step may be directly washed.

The washing step is generally classified into two types depending on the shape of the solid phase carrier. When the solid phase carrier has a particle shape like magnetic particles, a washing method involving dispersing magnetic particles in the washing solution may be given as an example. Meanwhile, when the solid phase carrier has a shape like a micro plate, a washing method involving bringing the surface thereof into contact with the washing solution may be given as an example. For any of those embodiments, it is preferred in the present invention to use, as the washing solution, a washing solution containing a surfactant. The surfactant is preferably one similar to the one that may be used in the complex forming step.

In addition, when the solid phase carrier is magnetic particles, the washing step preferably includes a magnetic collecting step of collecting the magnetic particles by magnetic force and separating the magnetic particles from a liquid phase, and a dispersing step of dispersing the magnetic particles that are separated by the magnetic collecting step in a washing solution. With this, unreacted substances or impurities in the biological sample can be more efficiently washed off, and separated and removed from the surfaces of the magnetic particles.

Specifically, the washing step may be performed by:
collecting the magnetic particles by applying a magnetic field to a reaction vessel to cause the magnetic particles to adhere to a wall of the reaction vessel; then removing the reaction supernatant; adding the washing solution as necessary; and
repeating an operation of similarly applying a magnetic field and removing the supernatant. The washing solution is preferably a washing solution which contains the surfactant and buffer solution described in the complex forming step.

### (Inorganic Salt or Organic Salt)

In the first separation method of the present invention, at least any one of the complex forming step and the washing step is performed in the presence of an inorganic salt or an organic salt at a final concentration of from 0.15 M to 2 M. From the viewpoint of the reactivity of the capturing reaction, it is preferred that the complex forming step be performed in the presence of an inorganic salt or an organic salt at a final concentration of from 0.15 M to 2 M. In addition, of the inorganic salt and the organic salt, the inorganic salt is preferred from the viewpoint of enhancing a desired effect of the present invention.

Examples of the inorganic salt include: an alkali metal halide; and an inorganic acid salt, such as a carbonate or a bicarbonate. Of those, an alkali metal halide is preferred, and an alkali metal chloride is more preferred. Specific examples of the inorganic salt include sodium chloride, potassium chloride, calcium chloride, sodium carbonate, and sodium bicarbonate, and those inorganic salts may be used alone or in combination thereof. Of those, sodium chloride, potassium chloride, and calcium chloride are preferred, sodium chloride and potassium chloride are more preferred, and sodium chloride is even more preferred.

As the organic salt, an organic acid salt is preferred, an alkali metal salt of an organic acid is more preferred, an alkali metal salt of a carboxylic acid is still more preferred, and a sodium salt of a carboxylic acid is even more preferred. Specific examples of the organic salt include sodium acetate, sodium citrate, sodium tartrate, sodium malate, sodium succinate, and sodium lactate, and those organic salts may be used alone or in combination thereof.

In addition, the final concentration of the inorganic salt or the organic salt in the system is from 0.15 M to 2 M. In each of the case where the final concentration as defined herein is less than 0.15 M and the case where the final concentration is more than 2 M, an increase in non-specific adsorption or a decrease in reactivity of the capturing reaction may occur, and in addition, there is also a risk of disruption of the vesicle owing to a difference in osmotic pressure between the inside and outside of the vesicle. The final concentration is preferably 0.175 M or more, more preferably 0.2 M or more, even more preferably 0.25 M or more, and is preferably 1 M or less, more preferably 0.5 M or less, still more preferably 0.45 M or less, even more preferably 0.35 M or less.

### (Dissociating Step)

The separation method of the present invention may also include, after the washing step, a dissociating step of dissociating the captured vesicle from the ligand.

As the condition for dissociation of a specific bond based on an antigen/antibody reaction, for a case in which the ligand is a specific antibody, various conditions have been known for affinity chromatography or the like (see, for example, "Afinity Chromatography Principles & Methods" Pharmacia LKB Biotechnology). The dissociating step may be performed in accordance with the condition. That is, a dissociating solution to be used in the present invention is exemplified by: an acidic solution, such as hydrochloric acid, sulfuric acid, propionic acid, acetic acid, or glycine/hydrochloric acid buffer; an alkali solution, such as an aqueous sodium hydroxide solution, an aqueous potassium hydroxide solution, an aqueous ammonia solution, or diethylamine; a high ionic strength solution, such as a 3 M aqueous solution of sodium chloride or a 4.5 M aqueous solution of magnesium chloride; a solution containing a surfactant, such as SDS, Triton X-100, or Tween 20; a buffer solution containing a substance for lowering polarity, such as dioxane or ethylene glycol; and a buffer solution containing urea, guanidine hydrochloride, or the like, in addition to chaotropic ions, such as trichloroacetic acid or thiocyanic acid.

### [Method of Detecting Nucleic Acid and Method of Detecting Protein]

The method of detecting a nucleic acid in a vesicle of the present invention further comprises a nucleic acid detecting step of detecting a nucleic acid in the vesicle after the first separation method.

In addition, a method of detecting a protein derived from a vesicle of the present invention further comprises a protein detecting step of detecting a protein present at least one of inside or on a surface of the vesicle after the first separation method.

Those detection methods may each be performed in accordance with a normal method except for performing the first separation method. When the vesicle having a lipid bilayer membrane is an exosome, the nucleic acid or the protein may be detected from a recovered exosome by a known method, such as a PCR method, an electrophoretic method, a western blot method, an immunochemical method, and flow cytometry. Of those, from the viewpoints of allowing detection to be performed simply and with good sensitivity, and allowing the three-dimensional structure of a native form to be maintained, flow cytometry is preferred. In addition, examples of the nucleic acid include miRNA and mRNA.

In particular, as the exosome is secreted from various cells, for example, cells of an immune system or various cancer cells, a nucleic acid (miRNA or the like) derived from an exosome can be detected by the nucleic acid detection method of the present invention, and by the analysis thereof, a physiological phenomenon or various diseases can be determined.

### [Signal Measurement Method]

A method of measuring a signal derived from a vesicle of the present invention further comprises a signal measuring step of measuring an intensity of a signal derived from the vesicle formed to have the complex after the first separation method.

The measurement of the intensity of the signal may be performed by an immunological measurement method, and examples thereof include enzyme immunoassay (EIA), an enzyme immunometric assay method (ELISA), fluorescent immunoassay (FIA), radioactive immunoassay (RIA), luminescence immunoassay, an immuno blotting method, a western blot method, and flow cytometry. Of those, from the viewpoints of allowing detection to be performed simply and with good sensitivity, and allowing the three-dimensional structure of a native form to be maintained, flow cytometry is preferred. In addition, from the viewpoint of allowing an antibody to be detected simply and with good sensitivity, an ELISA method is preferred. Examples of the ELISA method include a competition method and a sandwich method.

Now, an exemplary separation method for the case of using a sandwich ELISA method is described. First, a ligand which recognizes a surface antigen present on a vesicle surface is allowed to bind to a solid phase carrier, and then the resultant is brought into contact with a biological sample containing a vesicle having a lipid bilayer membrane to form a complex, followed by washing. A labeled antibody obtained by modifying a monoclonal antibody or an antibody fragment thereof, or a disease-specific membrane protein antibody or an antibody fragment thereof is further added to form an additional complex. Further, a label amount in the formed complex is detected. Thus, the amount of a signal derived from a vesicle contained in the biological sample or the amount of a disease-specific vesicle contained in the biological sample can be measured.

### [Disease Determination Method]

A method of determining an onset of a disease in a test subject of the present invention comprises a step of measuring, based on the above-mentioned signal measurement method, an intensity of a signal derived from the vesicle formed to have the complex by using a biological sample derived from a test subject (hereinafter sometimes referred to as step (I)).

Further, when the intensity of the signal measured in the step (I) is compared to the intensity of a signal of a biological sample derived from a control and the intensity of the signal from the test subject is recognized to be stronger than the intensity of the signal from the control, it may be determined that there is an onset of the disease in the test subject (hereinafter sometimes referred to as step (II)).

Examples of the disease which can be determined by the disease determination method of the present invention include a cancer disease (for example, colon cancer, breast cancer, endometrial cancer, cervical cancer, ovarian cancer, pancreas cancer, stomach cancer, esophageal cancer, liver cancer, lung cancer, kidney cancer, or skin cancer), an inflammation system related disease (for example, rheumatism, arthrosis deformans, nephropathy, pancreatitis, hepatitis, or allergic response), a neurodegenerative disease, such as Alzheimer disease, a brain disease, a disease related to immunodeficiency, infertility, a mental disease, such as depression or autism, an intractable disease, such as Parkinson's disease, an autoimmune disease, a circulatory system disease, a blood disease, a digestive system disease, a senile disease, and an infectious disease. Of those, the method is useful for determination of a cancer disease and a disease of an immunological system, and is particularly useful for determination of a cancer disease.

In addition, when applied to an index of immune activity of a cytotoxic T cell (CTL), the method can be used for determination of an effect of a cancer vaccine on a cancer disease.

The step (I) may be performed by a normal method except for performing the measurement by the above-mentioned signal measurement method.

In addition, in the step (II), comparison is made by performing a statistical analysis based on the signal of the biological sample derived from the control with respect to the intensity of the signal measured in the step (I). The analysis method is not particularly limited, and a known method may be used. In addition, in the determination thereafter, for example, when the signal of the biological sample derived from the test subject is stronger than the signal from the control, it is determined that there is a high possibility of having an onset of the disease. In the present invention, the control means an average person who belongs to the same age group of the same sex as the test subject and exhibits no onset of the disease. The intensity of the signal from the control may be measured at the same time with the intensity of the signal from the test subject, or a statistical value obtained from values that have been separately measured in advance may be used.

### [Method of Evaluating Drug Efficacy of Drug for Disease Treatment]

A method of evaluating drug efficacy of a drug for disease treatment of the present invention comprises a step of measuring, based on the above-mentioned signal measurement method, an intensity of a signal derived from the vesicle formed to have the complex by using a biological sample derived from a test subject before and after administration of a drug for disease treatment (hereinafter sometimes referred to as step (A)).

Further, when the intensity of the signal derived from the complex in the biological sample derived from the test subject after administration of the drug for disease treatment is recognized to be weaker than the intensity of the signal derived from the complex in the biological sample derived from the test subject before administration of the drug for disease treatment, it may be determined that there is a high possibility that the drug for disease treatment exhibits drug efficacy (hereinafter sometimes referred to as step (B)).

An example of the drug for disease treatment is a drug for treating a disease that can be determined by the above-mentioned determination method. Preferred specific examples thereof include an anti-cancer drug and a drug for an anti-immunological system related disease.

The step (A) may be performed by a normal method except for performing the measurement by the above-mentioned signal measurement method.

In addition, in the step (B), comparison is made by performing a statistical analysis based on the signal of the biological sample before administration of the drug for disease treatment with respect to the intensity of the signal measured in the step (A). The analysis method is not particularly limited, and a known method may be used. In addition, in the determination thereafter, for example, when a signal amount of the biological sample after administration of the drug for disease treatment is less than a signal amount of the biological sample before the administration, it is determined that the drug is highly likely to have an effect of inhibiting the disease.

In particular, as an exosome is secreted from various cells, for example, cells of an immune system or various cancer cells, it is believed that drug efficacy in a patient can be evaluated by measuring a change in a blood exosome before and after the administration of the drug for disease treatment (including a change in amount of a membrane protein in addition to an increase/decrease in presence amount of the exosome). In addition, for example, when a ligand for a membrane protein which is specific to a cancer cell is combined with a ligand for a surface antigen of an exosome, it is expected that, for example, specificity of cancer diagnosis can be improved or identification of cancer type can be made, and thus development of a diagnostic drug more specific to a cancer disease can be achieved.

### [Kit]

A kit of the present invention comprises: a solid phase carrier to which a ligand which recognizes a surface antigen present on a surface of a vesicle having a lipid bilayer membrane is bound; and a liquid composition containing an inorganic salt or an organic salt, in which a content of the inorganic salt or the organic salt is such an amount as to allow a final concentration thereof in a system to be adjusted to from 0.15 M to 2 M. Such kit is useful for the above-mentioned first separation method, determination of a disease, and evaluation of drug efficacy of a drug for disease treatment.

The solid phase carrier and the inorganic salt or the organic salt to be contained in the liquid composition may be similar to those used for the first separation method.

The final concentration is preferably 0.175 M or more, more preferably 0.2 M or more, even more preferably 0.25 M or more, and is preferably 1 M or less, more preferably 0.5 M or less, still more preferably 0.45 M or less, even more preferably 0.35 M or less.

Specifically, the content of the inorganic salt or the organic salt in the liquid composition is preferably 0.3 M or more, more preferably 0.4 M or more, still more preferably 0.5 M or more, and is preferably 4 M or less, more preferably 2 M or less, still more preferably 1 M or less, still more preferably 0.9 M or less, even more preferably 0.7 M or less.

In addition, the liquid composition may contain a surfactant similar to that in the first separation method. In addition, the pH of the liquid composition is not particularly limited, but the pH preferably falls within the range of from 5 to 10, and the pH more preferably falls within the range of from 6 to 8. In order to maintain a desired pH, a buffer solution is generally used, and examples thereof include a phosphate buffer solution, a tris(hydroxymethyl)aminomethane buffer solution, a HEPES buffer solution, and an MES buffer solution.

### [Liquid Composition, Diluent for specimen, and Second Separation Method]

A liquid composition of the present invention is a liquid composition to be used for a method of separating a vesicle having a lipid bilayer membrane including a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier, and a washing step of washing the complex, for being added in at least any one of the complex forming step and the washing step, the liquid composition comprising an inorganic salt or an organic salt, in which a content of the inorganic salt or the organic salt is such an amount as to allow a final concentration thereof in a system to be adjusted to from 0.15 M to 2 M.

In addition, a diluent for specimen of the present invention is a diluent for specimen to be used for a method of separating a vesicle having a lipid bilayer membrane from a biological sample containing the vesicle having a lipid bilayer membrane through use of an insoluble carrier, for forming a complex of the vesicle and the insoluble carrier, the diluent for specimen comprising an inorganic salt or an organic salt, in which a content of the inorganic salt or the organic salt is such an amount as to allow a final concentration thereof in a system to be adjusted to from 0.15 M to 2 M.

In addition, a second separation method of the present invention is a method of separating a vesicle having a lipid bilayer membrane from a biological sample containing the vesicle having a lipid bilayer membrane through use of an insoluble carrier, the method comprising adding, into a system, a diluent for specimen containing an inorganic salt or an organic salt, in which a content of the inorganic salt or the organic salt is such an amount as to allow a final concentration thereof in the system to be adjusted to from 0.15 M to 2 M.

The composition and pH of each of the liquid composition and the diluent for specimen of the present invention are the same as those of the liquid composition included in the kit of the present invention. In addition, the diluent for specimen of the present invention may be used for the separation of a vesicle using an insoluble carrier other than the solid phase carrier to which a ligand which recognizes a surface antigen present on a surface of the vesicle is bound (for example, a gel filtration carrier or a filter). Therefore, the diluent for specimen of the present invention can be used for the separation of a vesicle using various carriers.

### [Examples]

The present invention is hereinafter described in detail by way of Examples. The present invention is not limited to these Examples.

### (Test Example 1)

The non-specific adsorption reducing action of the reaction buffer was investigated by: mixing negative control particles, which had been obtained by binding anti mouse IgG antibody which did not react with an exosome to magnetic particles, a clinical specimen containing an exosome, and a reaction buffer; washing the resultant; and then detecting the exosome non-specifically adsorbing onto the negative control particles by ELISA using an antibody which recognized CD81 expressed on the membrane of the exosome. A specific procedure is described below.

First, the following reaction buffers were prepared as reaction buffers of Reference Example 1 and Examples 1 to 5.

Reaction buffers of Reference Example 1 and Examples 1 to 5: buffers each obtained by adding, to Tris buffered saline (TBS (10× TBS manufactured by Sigma-Aldrich diluted by a factor of 10, the same applies hereinafter)), polyoxyethylene (160) polyoxypropylene (30) glycol (Pluronic F-68, manufactured by Gibco, the same applies hereinafter) as a nonionic surfactant at a concentration of 0.03% (w/v), and further adding sodium chloride (manufactured by Wako Pure Chemical Industries, Ltd., JIS Special Grade) as an inorganic salt at a concentration shown in Table 2

**Table 2**

| | Inorganic salt |
|---|---|
| Reference Example 1 | NaCl |
| | 0.14 M |
| Example 1 | NaCl |
| | 0.3 M |
| Example 2 | NaCl |
| | 0.4 M |
| Example 3 | NaCl |
| | 0.5 M |
| Example 4 | NaCl |
| | 0.6 M |
| Example 5 | NaCl |
| | 0.7 M |

Subsequently, the following buffer was prepared as a control buffer.

Control buffer: a buffer obtained by adding, to TBS, polyoxyethylene (160) polyoxypropylene (30) glycol serving as a nonionic surfactant at a concentration of 0.03% (w/v)

In addition, the following test samples were prepared as test samples 1 to 5.
Test sample 1 (HT29 sup. in Control Buffer) : a test sample obtained by adding, to the control buffer, a 100× concentrate of HT29 cell culture supernatant at a dilution factor of 20 as a spike
Test sample 2 (Serum): pooled blood serum (manufactured by Kohjin Bio Co., Ltd.)
Test sample 3 (HT29 sup. in Serum) : a test sample obtained by adding, to pooled blood serum (manufactured by Kohj in Bio Co., Ltd.), a 100× concentrate of HT29 cell culture supernatant at a dilution factor of 20 as a spike
Test sample 4 (Plasma): pooled citrated blood plasma (manufactured by Kohjin Bio Co., Ltd.)
Test sample 5 (HT29 sup. in Plasma) : a test sample obtained by adding, to pooled citrated blood plasma (manufactured by Kohjin Bio Co., Ltd.), a 100× concentrate of HT29 cell culture supernatant at a dilution factor of 20 as a spike

Next, the following particles were prepared as negative control particles: particles obtained by binding anti mouse IgG antibody (manufactured by JSR Life Sciences Corporation, the same applies hereinafter) to magnetic particles (MS300/Carboxyl manufactured by JSR Life Sciences Corporation, the same applies hereinafter).

Subsequently, to a 96 well white plate (#23711007, manufactured by Corning Incorporated), 0.0125 mg of the negative control particles were added, and to each of the wells, 25 µL of the control buffer or any one of the test samples 1 to 5 was added. The reaction buffer of Reference Example 1 was added so that the final concentration of sodium chloride was 0.07 M, and a reaction was performed at 25°C for 20 minutes (pH: 7.4). In addition, a similar reaction was performed by using an equal amount of each of the reaction buffers of Examples 1 to 5 in place of the reaction buffer of Reference Example 1 (Example 1: final concentration of 0.15 M, Example 2: final concentration of 0.2 M, Example 3: final concentration of 0.25 M, Example 4: final concentration of 0.3 M, Example 5: final concentration of 0.35 M (Examples 1 to 5: pH 7.4)).

Next, the negative control particles after the reaction were washed with a washing buffer (TBS containing 0.1% by mass of Tween 20), and 50 µL of alkaline phosphatase (hereinafter abbreviated as ALP) labeled anti CD81 antibody (0.1 µg/mL) diluted with TBS containing 1% by mass of BSA was added, followed by a reaction at 25°C for 20 minutes. After washing with the same washing buffer as above, 50 µL of a luminescent substrate (Class III Series Lumipulse substrate solution, manufactured by Fujirebio Inc.) was added, and after 5 minutes, a luminescence intensity was measured by using a luminescence measuring device (GloMax, manufactured by Promega). The results are shown in FIG. 1-1 and FIG. 1-2.

As shown in FIG. 1-1 and FIG. 1-2, when the final concentration of sodium chloride was 0.07 M (Reference Example 1), a background signal attributed to non-specific adsorption of a test sample component was detected, but when the final concentration of sodium chloride was from 0.15 M to 0.35 M (Examples 1 to 5), the non-specific adsorption was reduced and the background signal was reduced.

### (Test Example 2-1)

Reactivity in the case of using each of the reaction buffers of Reference Example 1 and Examples 1 to 5 was evaluated by: preparing particles obtained by binding anti CD9 antibody (ab2215, manufactured by Abcam plc, the same applies hereinafter) to magnetic particles (hereinafter sometimes referred to as anti CD9 antibody bound magnetic particles); mixing the anti CD9 antibody bound magnetic particles, a clinical specimen containing an exosome, and each of the reaction buffers of Reference Example 1 and Examples 1 to 5; washing the resultant; and detecting the captured exosome by ELISA in the same manner as in Test Example 1. A specific procedure is described below.

First, the following test samples were prepared as test samples 6 to 8.
Test sample 6 (HT29 sup. in Control Buffer) : a test sample obtained by adding, to the control buffer produced in Test Example 1, a 100× concentrate of HT29 cell culture supernatant at a dilution factor of 100 as a spike
Test sample 7 (Serum): pooled blood serum (manufactured by Kohjin Bio Co., Ltd.)
Test sample 8 (HT29 sup. in Serum) : a test sample obtained by adding, to pooled blood serum (manufactured by Kohj in Bio Co., Ltd.), a 100× concentrate of HT29 cell culture supernatant at a dilution factor of 100 as a spike

Subsequently, to each of 2 mL Protein LoBind tubes (#0030108132, manufactured by Eppendorf), 0.1 mg of the anti CD9 antibody bound magnetic particles were added, and to each of the tubes, any one of the test samples 6 to 8 was added (test sample 6: 0.1 mL, test sample 7: 0.1 mL, test sample 8: 0.1 mL). The reaction buffer of Reference Example 1 was added so that the final concentration of sodium chloride was 0.07 M, and a reaction was performed at 25°C for 5 hours (pH: 7.4). In addition, a similar reaction was performed by using an equal amount of each of the reaction buffers of Examples 1 to 5 in place of the reaction buffer of Reference Example 1 (Example 1: final concentration of 0.15 M, Example 2: final concentration of 0.2 M, Example 3: final concentration of 0.25 M, Example 4: final concentration of 0.3 M, Example 5: final concentration of 0.35 M (Examples 1 to 5: pH 7.4)).

Next, the resultant was washed with 0.5 mL of Washing/Dilution Buffer (manufactured by JSR Life Sciences Corporation), and then dispensed into a 96 well white plate (#23711007, manufactured by Corning Incorporated) at 0.0125 mg/well. To the suspension, 50 µL of ALP labeled anti CD81 antibody (0.1 µg/mL) diluted with TBS containing 1% by mass of BSA was added, followed by a reaction at 25°C for 20 minutes. The particles after the reaction were washed with a washing buffer (TBS containing 0.1% by mass of Tween 20), and 50 µL of a luminescent substrate (Class III Series Lumipulse substrate solution, manufactured by Fujirebio Inc.) was added. After 5 minutes, a luminescence intensity was measured by using a luminescence measuring device (GloMax, manufactured by Promega). The results are shown in FIG. 2-1.

As shown in FIG. 2-1, it was found that when the final concentration of sodium chloride was from 0.15 M to 0.35 M (Examples 1 to 5), the reactivity of the capturing reaction in blood serum was enhanced.

### (Test Example 2-2)

The same test as that of Test Example 2-1 was performed except that the reaction temperature of the capturing reaction was changed from 25°C to 4°C. A specific procedure is described below.

To each of 2 mL Protein LoBind tubes (#0030108132, manufactured by Eppendorf), 0.1 mg of the anti CD9 antibody bound magnetic particles were added, and to each of the tubes, any one of the test samples 6 to 8 was added (test sample 6: 0.1 mL, test sample 7: 0.1 mL, test sample 8: 0.1 mL). The reaction buffer of Reference Example 1 was added so that the final concentration of sodium chloride was 0.07 M, and a reaction was performed at 4°C for 5 hours (pH: 7.4). In addition, a similar reaction was performed by using an equal amount of the reaction buffer of Example 3 in place of the reaction buffer of Reference Example 1 (Example 3: final concentration of 0.25 M, (pH: 7.4)).

Next, the resultant was washed with 0.5 mL of a washing buffer (TBS containing 0.1% by mass of Tween 20) and then dispensed into a 96 well white plate (#23711007, manufactured by Corning Incorporated) at 0.0125 mg/well. To the suspension, 50 µL of ALP labeled anti CD81 antibody (0.1 µg/mL) diluted with TBS containing 1% by mass of BSA was added, followed by a reaction at 25°C for 20 minutes. The particles after the reaction were washed with a washing buffer (TBS containing 0.1% by mass of Tween 20), and 50 µL of a luminescent substrate (Class III Series Lumipulse substrate solution, manufactured by Fujirebio Inc.) was added. After 5 minutes, a luminescence intensity was measured by using a luminescence measuring device (GloMax, manufactured by Promega). The results are shown in FIG. 2-2. In FIG. 2-2, the results of the test performed at a reaction temperature of 25°C are also shown.

It was found from the results of Test Example 2-2 (FIG. 2-2) that, when the final concentration of sodium chloride was 0.25 M (Example 3), the reactivity of the capturing reaction in blood serum was satisfactory irrespective of whether the reaction temperature was 4°C or 25°C.

### (Test Example 3)

The non-specific adsorption reducing action of the reaction buffer of Example 3 was investigated for different test samples. A specific procedure is described below.

First, the following test samples were prepared as test samples 9 to 17.
Test sample 9 (HT29 sup. spike Control Buffer): a test sample obtained by adding, to the control buffer produced in Test Example 1, a 100× concentrate of HT29 cell culture supernatant at a dilution factor of 100 as a spike
Test sample 10 (Serum): pooled blood serum (manufactured by Kohjin Bio Co., Ltd.)
Test sample 11 (HT29 sup. spike Serum): a test sample obtained by adding, to pooled blood serum (manufactured by Kohj in Bio Co., Ltd.), a 100× concentrate of HT29 cell culture supernatant at a dilution factor of 100 as a spike
Test sample 12 (Plasma (Heparin)): pooled heparin blood plasma (manufactured by Kohjin Bio Co., Ltd.)
Test sample 13 (HT29 sup. spike Plasma (Heparin)): a test sample obtained by adding, to pooled heparin blood plasma (manufactured by Kohjin Bio Co., Ltd.), a 100× concentrate of HT29 cell culture supernatant at a dilution factor of 100 as a spike
Test sample 14 (Plasma (EDTA)): pooled EDTA blood plasma (manufactured by Kohjin Bio Co., Ltd.)
Test sample 15 (HT29 sup. spike Plasma (EDTA)): a test sample obtained by adding, to pooled EDTA blood plasma (manufactured by Kohjin Bio Co., Ltd.), a 100× concentrate of HT29 cell culture supernatant at a dilution factor of 100 as a spike
Test sample 16 (Plasma (Citrate)): pooled citrated blood plasma (manufactured by Kohjin Bio Co., Ltd.)
Test sample 17 (HT29 sup. spike Plasma (Citrate)): a test sample obtained by adding, to pooled citrated blood plasma (manufactured by Kohjin Bio Co., Ltd.), a 100× concentrate of HT29 cell culture supernatant at a dilution factor of 100 as a spike

Subsequently, to each of 2 mL Protein LoBind tubes (#0030108132, manufactured by Eppendorf), 0.1 mg of the negative control particles produced in Test Example 1 were added, and to each of the tubes, any one of the test samples 9 to 17 was added at 0.1 mL. The reaction buffer of Reference Example 1 was added so that the final concentration of sodium chloride was 0.07 M, and a reaction was performed at 25°C for 5 hours (pH: 7.4). In addition, a similar reaction was performed by using an equal amount of the reaction buffer of Example 3 in place of the reaction buffer of Reference Example 1 (Example 3: final concentration of 0.25 M (pH: 7.4)).

Next, the resultant was washed with 0.5 mL of a washing buffer (TBS containing 0.1% by mass of Tween 20), and then dispensed into a 96 well white plate (#23711007, manufactured by Corning Incorporated) at 0.0125 mg/well. To the suspension, 50 µL of ALP labeled anti CD81 antibody (0.1 µg/mL) diluted with TBS containing 1% by mass of BSA was added, followed by a reaction at 25°C for 20 minutes. The particles after the reaction were washed with a washing buffer (TBS containing 0.1% by mass of Tween 20), and 50 µL of a luminescent substrate (Class III Series Lumipulse substrate solution, manufactured by Fujirebio Inc.) was added. After 5 minutes, a luminescence intensity was measured by using a luminescence measuring device (GloMax, manufactured by Promega).

As shown in FIG. 3, when the final concentration of sodium chloride was 0.25 M (Example 3), the background signal was small irrespective of the kind of the test sample.

### (Test Example 4)

The reactivity enhancing action of the reaction buffer of Example 3 was investigated for different test samples. A specific procedure is described below.

To each of 2 mL Protein LoBind tubes (#0030108132, manufactured by Eppendorf), 0.1 mg of the anti CD9 antibody bound magnetic particles were added, and to each of the tubes, any one of the test samples 9 to 13 described in Test Example 3 was added at 0.1 mL. The reaction buffer of Reference Example 1 was added so that the final concentration of sodium chloride was 0.07 M, and a reaction was performed at 25°C for 5 hours (pH: 7.4). In addition, a similar reaction was performed by using an equal amount of the reaction buffer of Example 3 in place of the reaction buffer of Reference Example 1 (Example 3: final concentration of 0.25 M (pH: 7.4)).

Next, the resultant was washed with 0.5 mL of a washing buffer (TBS containing 0.1% by mass of Tween 20), and then dispensed into a 96 well white plate (#23711007, manufactured by Corning Incorporated) at 0.0125 mg/well. To the suspension, 50 µL of ALP labeled anti CD81 antibody (0.1 µg/mL) diluted with TBS containing 1% by mass of BSA was added, followed by a reaction at 25°C for 20 minutes. The particles after the reaction were washed with a washing buffer (TBS containing 0.1% by mass of Tween 20), and 50 µL of a luminescent substrate (Class III Series Lumipulse substrate solution, manufactured by Fujirebio Inc.) was added. After 5 minutes, a luminescence intensity was measured by using a luminescence measuring device (GloMax, manufactured by Promega). The results are shown in FIG. 4.

As shown in FIG. 4, when the final concentration of sodium chloride was 0.25 M (Example 3), the reactivity of the capturing reaction was high irrespective of the kind of the test sample.

### (Test Example 5)

The following particles were prepared: particles obtained by binding anti CD63 antibody (manufactured by JSR Life Sciences Corporation) to magnetic particles (hereinafter referred to as anti CD63 antibody bound magnetic particles) ; particles obtained by binding anti CD81 antibody (Clone M38, Abnova MAB6435) to magnetic particles (hereinafter referred to as anti CD81 antibody bound magnetic particles) ; and particles obtained by binding anti EpCAM antibody (manufactured by JSR Life Sciences Corporation) to magnetic particles (hereinafter referred to as anti EpCAM antibody bound magnetic particles).

The reactivity enhancing action of the reaction buffer of Example 3 was investigated in the same manner as in Test Example 4 except that: the anti CD9 antibody bound magnetic particles were changed to the anti CD63 antibody bound magnetic particles, the anti CD81 antibody bound magnetic particles, or the anti EpCAM antibody bound magnetic particles; and the test samples 10 to 13 described in Test Example 3 were used as the test samples. The results are shown in FIG. 5-1 (anti CD63 antibody bound magnetic particles), FIG. 5-2 (anti CD81 antibody bound magnetic particles), and FIG. 5-3 (anti EpCAM antibody bound magnetic particles).

### (Test Example 6 - Detection of Nucleic Acid in Vesicle)

Twelve 2 mL Protein LoBind tubes (#0030108132, manufactured by Eppendorf) were prepared and numbered as tubes 1 to 12. 0.1 1 mg of the anti CD9 antibody bound magnetic particles were added to each of the tubes 1 to 4, 0.5 mg of the anti CD9 antibody bound magnetic particles were added to each of the tubes 5 to 8, and 1 mg of the anti CD9 antibody bound magnetic particles were added to each of the tubes 9 to 12.

Subsequently, to each of the tubes 1 to 12, 1 mL of the reaction buffer of Example 3 was added so that the final concentration of sodium chloride was 0.25 M, and 1 mL of the test sample 10 described in Test Example 3 was further added. A reaction was performed at 25°C for 1 hour in each of the tubes 1, 5, and 9, at 25°C for 5 hours in each of the tubes 2, 6, and 10, at 25°C for 24 hours in each of the tubes 3, 7, and 11, or at 4°C for 24 hours in each of the tubes 4, 8, and 12.

Next, the particles after the reaction in each tube were washed 3 times with 0.5 mL of a washing buffer (TBS containing 0.1% by mass of Tween 20), and 0.5 mL of the resultant suspension was dispensed into a fresh 2 mL Protein LoBind tube (#0030108132, manufactured by Eppendorf) different from those described above. The antibody bound magnetic particles were collected by magnetic collecting through use of a magnetic collection rack, and then 0.4 mL of the supernatant was removed. From 0.1 mL of the remaining antibody bound particle liquid, microRNA was recovered and quantified. The results are shown in FIG. 6.

The recovery of microRNA was performed using ExoCap™ Nucleic Acid Elution Buffer (#MEX-E, manufactured by MBL). In addition, the quantification of microRNA (miR-21) was performed using TaqMan MicroRNA Reverse Transcription Kit (#4366597, manufactured by Life Technologies Corporation), TaqMan MicroRNA Assays (#186948384, manufactured by Life Technologies Corporation), and TaqMan Universal Master Mix II, no UNG (#4440040, manufactured by Life Technologies Corporation), and the operation was performed in accordance with the recommended protocol.

As shown in FIG. 6, the nucleic acid in the vesicle derived from healthy human blood serum was able to be detected by performing the reaction in the presence of sodium chloride at a final concentration of 0.25 M. Further, it was confirmed that the amount of the nucleic acid detected increased in a manner dependent on the amount of the antibody bound particles, temperature, and time.

### (Test Example 7 - Detection of Nucleic Acid in Vesicle)

Eight 2 mL Protein LoBind tubes (#0030108132, manufactured by Eppendorf) were prepared and numbered as tubes 13 to 20. 1 mg of the anti CD9 antibody bound magnetic particles were added to each of the tubes 13 and 14, 1 mg of the anti CD63 antibody bound magnetic particles were added to each of the tubes 15 and 16, 1 mg of the anti CD81 antibody bound magnetic particles were added to each of the tubes 17 and 18, and 1 mg of the composite antibody bound magnetic particles (obtained by mixing the anti CD9 antibody bound magnetic particles, the anti CD63 antibody bound magnetic particles, the anti CD81 antibody bound magnetic particles, and the anti EpCAM antibody bound magnetic particles at a ratio of 1:1:1:1) were added to each of the tubes 19 and 20.

Subsequently, to each of the tubes 13 to 20, 0.3 mL of the reaction buffer of Example 3 was added so that the final concentration of sodium chloride was 0.25 M. 0.3 mL of the test sample 10 described in Test Example 3 was added to each of the odd-numbered tubes, and 0.3 mL of the test sample 12 described in Test Example 3 was added to each of the even-numbered tubes, followed by a reaction at 25°C for 24 hours.

Next, the particles after the reaction in each tube were washed 3 times with 0.5 mL of a washing buffer (TBS containing 0.1% by mass of Tween 20), and 0.5 mL of the resultant suspension was dispensed into a fresh 2 mL Protein LoBind tube (#0030108132, manufactured by Eppendorf) different from those described above. The antibody bound magnetic particles were collected by magnetic collecting through use of a magnetic collection rack, and then 0.4 mL of the supernatant was removed. From 0.1 mL of the remaining antibody bound particle liquid, microRNA was recovered and quantified. The results are shown in FIG. 7-1 and FIG. 7-2.

The recovery of microRNA was performed using miRNeasy Serum/Plasma Kit (50) (#217184, manufactured by QIAGEN). In addition, the quantification of microRNA (miR-21) was performed using TaqMan MicroRNA Reverse Transcription Kit (#4366597, manufactured by Life Technologies Corporation), TaqMan MicroRNA Assays (#186948384, manufactured by Life Technologies Corporation), and TaqMan Universal Master Mix II, no UNG (#4440040, manufactured by Life Technologies Corporation), and the operation was performed in accordance with the recommended protocol.

In addition, for comparison, the detection of a nucleic acid in a vesicle was performed by using an ultracentrifugation method as a standard method of recovering an exosome. Specifically, 11 mL of each of the test samples 10 and 12 described in Test Example 3 was centrifuged using an ultracentrifuge (Optima XE-90, manufactured by Beckman Coulter) at 100,000×g and 4°C for 70 minutes. The supernatant was removed, and the precipitate was suspended by adding 11 mL of PBS. The resultant was centrifuged again using an ultracentrifuge (Optima XE-90, manufactured by Beckman Coulter) at 100,000 rpm and 4°C for 70 minutes. The supernatant was removed, and the precipitate was suspended by adding 0.11 mL of PBS. 3 µL of the suspension was used for the recovery and quantification of microRNA. The results are shown in FIG. 7-1 and FIG. 7-2.

The recovery of microRNA and the quantification of microRNA (miR-21) were performed in the same manner as above.

As shown in FIG. 7-1, it was confirmed that the nucleic acid in the vesicle derived from healthy human blood serum was able to be detected at a level comparable to that of the ultracentrifugation method by performing the reaction in the presence of sodium chloride at a final concentration of 0.25 M.

As shown in FIG. 7-2, the nucleic acid in the vesicle derived from healthy human heparin blood plasma was not able to be detected when the ultracentrifugation method was used. This is presumably because healthy human heparin blood plasma is considered to generally contain a substance that causes PCR inhibition, and the substance that inhibits PCR remains in a sample recovered by the ultracentrifugation method. Meanwhile, when the reaction was performed in the presence of sodium chloride at a final concentration of 0.25 M, the nucleic acid in the vesicle derived from healthy human heparin blood plasma was able to be detected.

As apparent from those results, the nucleic acid in the vesicle was able to be detected irrespective of the kind of the test sample, such as blood serum or blood plasma, by subjecting the vesicle and the particles to the reaction in the presence of sodium chloride at a final concentration of 0.25 M.

### (Test Example 8 - Detection of Protein on Vesicle Surface)

Ten 2 mL Protein LoBind tubes (#0030108132, manufactured by Eppendorf) were prepared and numbered as tubes 21 to 30. To each of the tubes, 0.2 mg of magnetic particles were added as shown below. 0.2 mg of the anti CD9 antibody bound magnetic particles were added to each of the tubes 21 and 22, 0.2 mg of the anti CD63 antibody bound magnetic particles were added to each of the tubes 23 and 24, 0.2 mg of the anti CD81 antibody bound magnetic particles were added to each of the tubes 25 and 26, 0.2 mg of the anti EpCAM antibody bound magnetic particles were added to each of the tubes 27 and 28, and 0.2 mg of the composite antibody bound magnetic particles (obtained by mixing the anti CD9 antibody bound magnetic particles, the anti CD63 antibody bound magnetic particles, the anti CD81 antibody bound magnetic particles, and the anti EpCAM antibody bound magnetic particles at a ratio of 1:1:1:1) were added to each of the tubes 29 and 30.

Subsequently, to each of the tubes 21 to 30, 1.0 mL of the reaction buffer of Example 3 was added so that the final concentration of sodium chloride was 0.25 M. 1.0 mL of the test sample 10 described in Test Example 3 was added to each of the odd-numbered tubes, and 1.0 mL of the test sample 12 described in Test Example 3 was added to each of the even-numbered tubes, followed by a reaction at 25°C for 3 hours.

Next, the particles after the reaction in each tube were washed 3 times with 0.5 mL of a washing buffer (TBS containing 0.1% by mass of Tween 20), and 0.5 mL of the resultant suspension was dispensed into a fresh 2 mL Protein LoBind tube (#0030108132, manufactured by Eppendorf) different from those described above.

Subsequently, after discarding the washing solution by magnetic collecting, the antibody bound magnetic particles were suspended in 20 µL of a 1× sample buffer and allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer (TBS containing 0.1% (w/v) Tween 20).

The resultant was subjected to a reaction with anti CD9 antibody, anti CD63 antibody, or anti CD81 antibody serving as a primary antibody and a reaction with HRP labeled anti mouse IgG antibody (Mouse TrueBlot ULTRA: Anti-Mouse IgG HRP, Rockland 18-8817-33) serving as a labeled antibody, each at 25°C for 1 hour. The resultant was washed with a washing buffer (TBS containing 0.1% (w/v) Tween 20), and then subjected to a reaction with a luminescent substrate. A western blot image was observed with a luminescence measuring device (LAS-3000, FUJIFILM). The results are shown in FIG. 8-1 and FIG. 8-2.

In addition, for comparison, the detection of a protein on a surface of a vesicle was performed by using an ultracentrifugation method as a standard method of recovering an exosome. Specifically, 11 mL of each of the test samples 10 and 12 described in Test Example 3 was centrifuged using an ultracentrifuge (Optima XE-90, manufactured by Beckman Coulter) at 100, 000×g and 4°C for 70 minutes. The supernatant was removed, and the precipitate was suspended by adding 11 mL of PBS. The resultant was centrifuged again using an ultracentrifuge (Optima XE-90, manufactured by Beckman Coulter) at 100, 000×g and 4°C for 70 minutes. The supernatant was removed, and the precipitate was suspended by adding 0.11 mL of PBS. To 10 µL of the suspension, 5 µL of a 4× sample buffer and 5 µL of PBS were added, and the resultant was allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer (TBS containing 0.1% (w/v) Tween 20).

The resultant was subjected to a reaction with anti CD9 antibody, anti CD63 antibody, or anti CD81 antibody serving as a primary antibody and a reaction with HRP labeled anti mouse IgG antibody (Mouse TrueBlot ULTRA: Anti-Mouse IgG HRP, Rockland 18-8817-33) serving as a labeled antibody, each at 25°C for 1 hour. The resultant was washed with a washing buffer (TBS containing 0.1% (w/v) Tween 20), and then subjected to a reaction with a luminescent substrate. A western blot image was observed with a luminescence measuring device (LAS-3000, FUJIFILM). The results are shown in FIG. 8-1 and FIG. 8-2.

As shown in FIG. 8-1 and FIG. 8-2, it was confirmed that the protein on the surface of the vesicle derived from each of healthy human blood serum and healthy human heparin blood plasma was able to be detected at a level comparable to that of the ultracentrifugation method by subjecting the vesicle and the particles to the reaction in the presence of sodium chloride at a final concentration of 0.25 M. In addition, when the anti EpCAM antibody bound magnetic particles were used, no vesicle surface protein was detected. EpCAM protein is hardly present in blood of a healthy person and its blood concentration is considered to increase owing to a disease, such as cancer. It was confirmed that non-specific reaction was able to be suppressed by subjecting the vesicle and the particles to the reaction in the presence of sodium chloride at a final concentration of 0.25 M.

### (Test Example 9-1 - Detection of Protein Maintaining Three-dimensional Structure of Native Form on Vesicle Surface (1))

Eight 2 mL Protein LoBind tubes (#0030108132, manufactured by Eppendorf) were prepared and numbered as tubes 31 to 38. 0.1 mg of the anti CD9 antibody bound magnetic particles were added to each of the tubes 31 and 32, 0.1 mg of the anti CD63 antibody bound magnetic particles were added to each of the tubes 33 and 34, 0.1 mg of the anti CD81 antibody bound magnetic particles were added to each of the tubes 35 and 36, and 0.1 mg of the anti EpCAM antibody bound magnetic particles were added to each of the tubes 37 and 38. 0.3 mL of the reaction buffer of Example 3 was added to each of the tubes 31 to 38 so that the final concentration of sodium chloride was 0.25 M.

Subsequently, to each of the tubes 31, 33, 35, and 37, 0.3 mL of the test sample 10 described in Test Example 3 was added, followed by a reaction at 25°C overnight. In addition, to each of the tubes 32, 34, 36, and 38, 0.3 mL of the test sample 12 described in Test Example 3 was added, followed by a reaction at 25°C overnight.

Next, the particles after the reaction in each tube were washed 2 times with 0.25 mL of a washing buffer (TBS containing 0.1% by mass of Tween 20), and after magnetic collecting, the supernatant was removed, followed by suspension in 1 mL of phosphate buffered saline.

40 fresh 2 mL Protein LoBind tubes (#0030108132, manufactured by Eppendorf) different from those described above were prepared and numbered as tubes 39 to 78. 0.1 mL of the suspension in the tube 31 was dispensed into each of the tubes 39 to 43. 0.1 mL of the suspension in the tube 32 was dispensed into each of the tubes 44 to 48. 0.1 mL of the suspension in the tube 33 was dispensed into each of the tubes 49 to 53. 0.1 mL of the suspension in the tube 34 was dispensed into each of the tubes 54 to 58. 0.1 mL of the suspension in the tube 35 was dispensed into each of the tubes 59 to 63. 0.1 mL of the suspension in the tube 36 was dispensed into each of the tubes 64 to 68. 0.1 mL of the suspension in the tube 37 was dispensed into each of the tubes 69 to 73. 0.1 mL of the suspension in the tube 38 was dispensed into each of the tubes 74 to 78.

Subsequently, 5 µL of phycoerythrin (hereinafter abbreviated as PE) labeled anti mouse IgG antibody (manufactured by SONY) was added as a control to each of the tubes 39, 44, 49, 54, 59, 64, 69, and 74, 5 µL of PE labeled anti CD9 antibody (manufactured by SONY) was added to each of the tubes 40, 45, 50, 55, 60, 65, 70, and 75, 5 µL of PE labeled anti CD63 antibody (manufactured by SONY) was added to each of the tubes 41, 46, 51, 56, 61, 66, 71, and 76, 5 µL of PE labeled anti CD81 antibody (manufactured by SONY) was added to each of the tubes 42, 47, 52, 57, 62, 67, 72, and 77, and 5 µL of PE labeled anti EpCAM antibody (manufactured by SONY) was added to each of the tubes 43, 48, 53, 58, 63, 68, 73, and 78. After that, the tubes 39 to 78 were suspended under the conditions of 25°C and 1,000 rpm for 1 hour.

Next, the particles after the reaction in each tube were washed 2 times with 0.5 mL of phosphate buffered saline, and then a fluorescence signal of each sample was observed using a flow cytometer (BD Accuri C6, manufactured by BD). The results are shown in FIG. 9-1 and FIG. 9-2.

As shown in FIG. 9-1 and FIG. 9-2, it was confirmed that CD9, CD63, and CD81 on the surface of the vesicle derived from each of healthy human blood serum and healthy human heparin blood plasma were able to be detected by subjecting the vesicle and the particles to the reaction in the presence of sodium chloride at a final concentration of 0.25 M.

In addition, when the anti CD9 antibody bound magnetic particles, the anti CD63 antibody bound magnetic particles, or the anti CD81 antibody bound magnetic particles were used, the peak of EpCAM, which was considered to be expressed by oncogenesis, detected with the anti EpCAM antibody did not show a peak shift as compared to that in the case of the anti mouse IgG antibody serving as a control. It is considered that EpCAM is not detected from a test sample from a healthy person.

Further, when the anti EpCAM antibody bound magnetic particles were used as a solid phase carrier, the peak detected with the anti mouse IgG antibody did not differ very much from the peak detected with the anti CD9 antibody, the anti CD63 antibody, the anti CD81 antibody, or the anti EpCAM antibody. That is, when the anti EpCAM antibody bound magnetic particles were used as a solid phase carrier, an exosome was not obtained from a test sample from a healthy person. It is considered that non-specific adsorption is low.

In addition, the samples used for the analysis were not treated with heat, a chemical, or the like changing the three-dimensional structure of a protein, and hence it is considered that CD9, CD63, and CD81 of native forms present on the vesicle surface were able to be detected.

### (Test Example 9-2 - Detection of Protein Maintaining Three-dimensional Structure of Native Form on Vesicle Surface (2))

Prior to the test, test samples 18 and 19 described below were prepared.
Test sample 18 (HT29 sup. spike Serum): a test sample obtained by adding, to pooled blood serum (manufactured by Kohj in Bio Co., Ltd.), a 100× concentrate of HT29 cell culture supernatant at a dilution factor of 10 as a spike
Test sample 19 (HT29 sup. spike Plasma (Heparin)): a test sample obtained by adding, to pooled heparin blood plasma (manufactured by Kohjin Bio Co., Ltd.), a 100× concentrate of HT29 cell culture supernatant at a dilution factor of 10 as a spike

Eight 2 mL Protein LoBind tubes (#0030108132, manufactured by Eppendorf) were prepared and numbered as tubes 79 to 86. 0.1 mg of the anti CD9 antibody bound magnetic particles were added to each of the tubes 79 and 80, 0.1 mg of the anti CD63 antibody bound magnetic particles were added to each of the tubes 81 and 82, 0.1 mg of the anti CD81 antibody bound magnetic particles were added to each of the tubes 83 and 84, and 0.1 mg of the anti EpCAM antibody bound magnetic particles were added to each of the tubes 85 and 86. 0.3 mL of the reaction buffer of Example 3 was added to each of the tubes 79 to 86 so that the final concentration of sodium chloride was 0.25 M.

Subsequently, to each of the tubes 79, 81, 83, and 85, 0.3 mL of the test sample 18 was added, followed by a reaction at 25°C overnight. In addition, to each of the tubes 80, 82, 84, and 86, 0.3 mL of the test sample 19 was added, followed by a reaction at 25°C overnight.

Next, the particles after the reaction in each tube were washed 2 times with 0.25 mL of a washing buffer (TBS containing 0.1% by mass of Tween 20), and after magnetic collecting, the supernatant was removed, followed by suspension in 1 mL of phosphate buffered saline.

40 fresh 2 mL Protein LoBind tubes (#0030108132, manufactured by Eppendorf) different from those described above were prepared and numbered as tubes 87 to 126. 0.1 mL of the suspension in the tube 79 was dispensed into each of the tubes 87 to 91. 0.1 mL of the suspension in the tube 80 was dispensed into each of the tubes 92 to 96. 0.1 mL of the suspension in the tube 81 was dispensed into each of the tubes 97 to 101. 0.1 mL of the suspension in the tube 82 was dispensed into each of the tubes 102 to 106. 0.1 mL of the suspension in the tube 83 was dispensed into each of the tubes 107 to 111. 0.1 mL of the suspension in the tube 84 was dispensed into each of the tubes 112 to 116. 0.1 mL of the suspension in the tube 85 was dispensed into each of the tubes 117 to 121. 0.1 mL of the suspension in the tube 86 was dispensed into each of the tubes 122 to 126.

Subsequently, 5 µL of PE labeled anti mouse IgG antibody (manufactured by SONY) was added as a control to each of the tubes 87, 92, 97, 102, 107, 112, 117, and 122, 5 µL of PE labeled anti CD9 antibody (manufactured by SONY) was added to each of the tubes 88, 93, 98, 103, 108, 113, 118, and 123, 5 µL of PE labeled anti CD63 antibody (manufactured by SONY) was added to each of the tubes 89, 94, 99, 104, 109, 114, 119, and 124, 5 µL of PE labeled anti CD81 antibody (manufactured by SONY) was added to each of the tubes 90, 95, 100, 105, 110, 115, 120, and 125, and 5 µL of PE labeled anti EpCAM antibody (manufactured by SONY) was added to each of the tubes 91, 96, 101, 106, 111, 116, 121, and 126. After that, the tubes 87 to 126 were suspended under the conditions of 25°C and 1,000 rpm for 1 hour.

Next, the particles after the reaction in each tube were washed 2 times with 0.5 mL of phosphate buffered saline, and then a fluorescence signal of each sample was observed using a flow cytometer (BD Accuri C6, manufactured by BD). The results are shown in FIG. 9-3 and FIG. 9-4.

As shown in FIG. 9-3 and FIG. 9-4, it was confirmed that CD9, CD63, and CD81 present on the surface of the vesicle derived from each of healthy human blood serum, healthy human heparin blood plasma, and human colon cancer HT29 cells were able to be detected by subjecting the vesicle and the particles to the reaction in the presence of sodium chloride at a final concentration of 0.25 M.

In addition, the peak of EpCAM, which was considered to be expressed by oncogenesis, detected with the anti EpCAM antibody showed a peak shift as compared to that in the case of the anti mouse IgG antibody serving as a control. This peak shift was not observed in FIG. 9-1 and FIG. 9-2, in which only test samples of healthy persons were used as samples. Thus, it has been found that the present invention can be used to determine a disease.

Further, when the anti EpCAM antibody bound magnetic particles were used as a solid phase carrier, the peak detected with the anti mouse IgG antibody did not differ very much from the peak detected with the anti CD9 antibody, the anti CD63 antibody, the anti CD81 antibody, or the anti EpCAM antibody. That is, when the anti EpCAM antibody bound magnetic particles were used as a solid phase carrier, an exosome was not obtained from a test sample from a healthy person. It is considered that non-specific adsorption is low.

In addition, the samples used for the analysis were not treated with heat, a chemical, or the like changing the three-dimensional structure of a protein, and hence it is considered that CD9, CD63, and CD81 of native forms present on the vesicle surface were able to be detected.

### (Test Example 10)

Twenty 2 mL Protein LoBind tubes (#0030108132, manufactured by Eppendorf) were prepared and numbered as tubes 127 to 146. 0.2 mg of the anti CD9 antibody bound magnetic particles were added to each of the tubes 127 to 130, 0.2 mg of the anti CD63 antibody bound magnetic particles were added to each of the tubes 131 to 134, 0.2 mg of the anti CD81 antibody bound magnetic particles were added to each of the tubes 135 to 138, 0.2 mg of the anti EpCAM antibody bound magnetic particles were added to each of the tubes 139 to 142, and 0.2 mg of the composite antibody bound magnetic particles (obtained by mixing the anti CD9 antibody bound magnetic particles, the anti CD63 antibody bound magnetic particles, the anti CD81 antibody bound magnetic particles, and the anti EpCAM antibody bound magnetic particles at a ratio of 1:1:1:1) were added to each of the tubes 143 to 146.

Subsequently, to each of the tubes 127 to 146, 0.1 mL of the reaction buffer of Example 3 was added so that the final concentration of sodium chloride was 0.25 M. 0.1 mL of the test sample 10 described in Test Example 3 was added to each of the tubes 127, 131, 135, 139, and 143, 0.1 mL of the test sample 18 described in Test Example 9-2 was added to each of the tubes 128, 132, 136, 140, and 144, 0.1 mL of the test sample 12 described in Test Example 3 was added to each of the tubes 129, 133, 137, 141, and 145, and 0.1 mL of the test sample 19 described in Test Example 9-2 was added to each of the tubes 130, 134, 138, 142, and 146, followed by a reaction at 25°C for 24 hours.

Next, the particles after the reaction in each tube were washed 3 times with 0.5 mL of a washing buffer (TBS containing 0.1% by mass of Tween 20), and 0.5 mL of the resultant suspension was dispensed into a fresh 2 mL Protein LoBind tube (#0030108132, manufactured by Eppendorf) different from those described above.

Subsequently, after discarding the washing solution by magnetic collecting, the antibody bound magnetic particles were suspended in 20 µL of a 1× sample buffer and allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer (TBS containing 0.1% (w/v) Tween 20).

The resultant was subjected to a reaction with anti CD9 antibody, anti EpCAM antibody, or anti Alix antibody serving as a primary antibody and a reaction with HRP labeled anti mouse IgG antibody (Mouse TrueBlot ULTRA: Anti-Mouse IgG HRP, Rockland 18-8817-33) serving as a labeled antibody, each at 25°C for 1 hour. The resultant was washed with a washing buffer (TBS containing 0.1% (w/v) Tween 20), and then subjected to a reaction with a luminescent substrate. A western blot image was observed with a luminescence measuring device (LAS-3000, FUJIFILM). The results are shown in FIG. 10-1 and FIG. 10-2.

In addition, for comparison, the detection of a protein on a surface of a vesicle was performed by using an ultracentrifugation method as a standard method of recovering an exosome. Specifically, 11 mL of each of the test samples 10, 12, 18, and 19 was centrifuged using an ultracentrifuge (Optima XE-90, manufactured by Beckman Coulter) at 100,000×g and 4°C for 70 minutes. The supernatant was removed, and the precipitate was suspended by adding 11 mL of PBS. The resultant was centrifuged again using an ultracentrifuge (Optima XE-90, manufactured by Beckman Coulter) at 100,000×g and 4°C for 70 minutes. The supernatant was removed, and the precipitate was suspended by adding 0.11 mL of PBS. To 1 µL of the suspension, 5 µL of a 4× sample buffer and 14 µL of PBS were added, and the resultant was allowed to stand at 95°C for 5 minutes. The entire amount (20 µL) of each sample was applied and subjected to SDS-PAGE. The gel was transferred to a PVDF membrane, and then shaken at 37°C for 2 hours with a blocking buffer (TBS containing 1% (w/v) BSA and 0.1% (w/v) Tween 20). The resultant was washed with a washing buffer (TBS containing 0.1% (w/v) Tween 20).

The resultant was subjected to a reaction with anti CD9 antibody, anti EpCAM antibody, or anti Alix antibody serving as a primary antibody and a reaction with HRP labeled anti mouse IgG antibody (Mouse TrueBlot ULTRA: Anti-Mouse IgG HRP, Rockland 18-8817-33) serving as a labeled antibody, each at 25°C for 1 hour. The resultant was washed with a washing buffer (TBS containing 0.1% (w/v) Tween 20), and then subjected to a reaction with a luminescent substrate. A western blot image was observed with a luminescence measuring device (LAS-3000, FUJIFILM). The results are shown in FIG. 10-1 and FIG. 10-2.

As shown in FIG. 10-1 and FIG. 10-2, it was confirmed that the protein on the surface of the vesicle derived from each of healthy human blood serum, healthy human heparin blood plasma, and human colon cancer HT29 cells was able to be detected at a level comparable to or higher than that of the ultracentrifugation method by subjecting the vesicle and the particles to the reaction in the presence of sodium chloride at a final concentration of 0.25 M. In addition, in the case of detection using the anti EpCAM antibody, no band was detected for healthy human blood serum or heparin blood plasma, but when the culture supernatant of the human colon cancer HT29 cells was added thereto, a band was detected. Thus, it has been found that the present invention can be used to determine a disease.

## Claims

1. A method of separating a vesicle having a lipid bilayer membrane, comprising:
a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier; and
a washing step of washing the complex,
wherein at least any one of the complex forming step and the washing step is performed under an environment having a salt concentration of from 0.15 M to 2 M.

2. The separation method according to claim 1, wherein the complex forming step is performed under an environment having a salt concentration of from 0.15 M to 2 M.

3. The separation method according to claim 1 or 2, wherein the biological sample comprises body fluid or cell culture supernatant.

4. The separation method according to any one of claims 1 to 3, wherein the biological sample comprises body fluid.

5. The separation method according to any one of claims 1 to 4, wherein the complex forming step is performed using a complex forming reaction solution containing an inorganic salt.

6. The separation method according to claim 5, wherein the inorganic salt comprises an alkali metal halide.

7. The separation method according to any one of claims 1 to 6, wherein a complex forming reaction in the complex forming step is performed at a reaction temperature of from 2°C to 42°C.

8. The separation method according to any one of claims 1 to 7, wherein the ligand is an antibody which recognizes a surface antigen present on a surface of the vesicle.

9. The separation method according to any one of claims 1 to 8, wherein the vesicle is an exosome.

10. The separation method according to any one of claims 1 to 9, wherein the solid phase carrier is magnetic particles.

11. The separation method according to claim 10, wherein the washing step comprises a magnetic collecting step of collecting the magnetic particles by magnetic force and separating the magnetic particles from a liquid phase, and a dispersing step of dispersing the magnetic particles separated by the magnetic collecting step in a washing solution.

12. A method of detecting a nucleic acid in a vesicle, further comprising a nucleic acid detecting step of detecting a nucleic acid in the vesicle, after the separation method of any one of claims 1 to 11.

13. A method of detecting a protein derived from a vesicle, further comprising a protein detecting step of detecting a protein present at least one of inside or on a surface of the vesicle, after the separation method of any one of claims 1 to 11.

14. A method of measuring a signal derived from a vesicle, further comprising a signal measuring step of measuring an intensity of a signal derived from the vesicle formed to have the complex, after the separation method of any one of claims 1 to 11.

15. A method of determining an onset of a disease in a test subject, comprising a step of measuring, based on the signal measurement method of claim 14, an intensity of a signal derived from the vesicle formed to have the complex by using a biological sample derived from the test subject.

16. A method of evaluating drug efficacy of a drug for disease treatment, comprising a step of measuring, based on the signal measurement method of claim 14, an intensity of a signal derived from the vesicle formed to have the complex by using a biological sample derived from a test subject before and after administration of a drug for disease treatment.

17. A kit, comprising:
a solid phase carrier to which a ligand which recognizes a surface antigen present on a surface of a vesicle having a lipid bilayer membrane is bound; and
a liquid composition containing an inorganic salt or an organic salt and having a salt concentration of 0.15 M or more in terms of content of the inorganic salt or the organic salt.

18. The kit according to claim 17, which is used for determining a disease or evaluating drug efficacy of a drug for disease treatment.

19. A liquid composition to be used for a method of separating a vesicle having a lipid bilayer membrane, including a complex forming step of forming a complex of a vesicle having a lipid bilayer membrane and a solid phase carrier to which a ligand which recognizes a surface antigen present on a surface of the vesicle is bound, by bringing a biological sample containing the vesicle into contact with the solid phase carrier, and a washing step of washing the complex, for being added in at least any one of the complex forming step and the washing step,
the liquid composition having a salt concentration of 0.15 M or more.

20. A diluent for specimen to be used for a method of separating a vesicle having a lipid bilayer membrane from a biological sample containing the vesicle having a lipid bilayer membrane through use of an insoluble carrier, for forming a complex of the vesicle and the insoluble carrier,
the diluent for specimen having a salt concentration of 0.15 M or more.

21. The diluent for specimen according to claim 20, wherein the diluent for specimen comprises an inorganic salt.

22. The diluent for specimen according to claim 21, wherein the inorganic salt is an alkali metal halide.

23. A method of separating a vesicle having a lipid bilayer membrane from a biological sample containing the vesicle having a lipid bilayer membrane through use of an insoluble carrier,
the method comprising using a diluent for specimen having a salt concentration of 0.15 M or more.
